(19) Europäisches Patentamt
European Patent Office
Office européen des brevets

(11) Veröffentlichungsnummer: **0 089 028**
**B1**

(12) **EUROPÄISCHE PATENTSCHRIFT**

(45) Veröffentlichungstag der Patentschrift: **25.05.88**

(51) Int. Cl.$^4$: **C 07 D 473/08**, A 61 K 31/52

(21) Anmeldenummer: **83102422.9**

(22) Anmeldetag: **11.03.83**

(54) **Neue Theophyllin-Derivate und Verfahren zu ihrer Herstellung.**

(30) Priorität: **12.03.82 HU 76282**
**21.01.83 HU 76282**

(43) Veröffentlichungstag der Anmeldung:
**21.09.83 Patentblatt 83/38**

(45) Bekanntmachung des Hinweises auf die Patenterteilung:
**25.05.88 Patentblatt 88/21**

(84) Benannte Vertragsstaaten:
**AT BE CH DE FR GB IT LI NL SE**

(56) Entgegenhaltungen:
**DE-B-1 063 323**
**FR-A-2 480 286**
**GB-A-1 053 825**
**GB-A-1 187 348**
**US-A-4 197 300**

**IL FARMACO 36, Bd. 3 (1981), 201-219**

(73) Patentinhaber: **CHINOIN Gyogyszer és Vegyészeti Termékek Gyára RT.**
**To utca 1-5**
**H-1045 Budapest IV (HU)**

(72) Erfinder: **Korbonits, Dezsö, Dr. Dipl.-Ing. Chem.**
**Vérhalom u. 27/d**
**H-1025 Budapest (HU)**
Erfinder: **Szomor geb. Wundele, Mária Dipl.-Ing. Chem.**
**Fö u. 21**
**H-1011 Budapest (HU)**
Erfinder: **Héja, Gergely, Dr. Dipl.-Ing. Chem.**
**Sollner ut 27**
**H-1131 Budapest (HU)**
Erfinder: **Szvoboda geb. Kanzel, Ida**
**Váci M. ut 21**
**H-2120 Dunakeszi (HU)**
Erfinder: **Kiss, Pál, Dr. Dipl.-Ing. Chem.**
**Vetés ut 82**
**H-1046 Budapest (HU)**
Erfinder: **Gönczi, Csaba, Dr. Dipl.-Ing. Chem.**
**Lisznyai ut 5**
**H-1016 Budapest (HU)**
Erfinder: **Pálosi, Endre, Dipl.-Ing. Chem.**
**Pasaréti ut 114/a**
**H-1026 Budapest (HU)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**EP 0 089 028 B1**

(72) Erfinder: **Kovács, Gábor, Dr. Dipl.-Ing. Chem.**
**Róna-park 4**
**H-1042 Budapest (HU)**
Erfinder: **Kun, Judit, Dipl.-Ing. Chem.**
**Gyenes ut 17**
**H-1032 Budapest (HU)**
Erfinder: **Minker, Emil, Dr.**
**Somogyi ut 6**
**H-6701 Szeged (HU)**
Erfinder: **Virág, Sándor, Dr.**
**Sallai Imre u. 29/a**
**H-1136 Budapest (HU)**
Erfinder: **Sebestyén, Gyula, Dr.**
**Biro Lajos u. 29**
**H-1089 Budapest (HU)**
Erfinder: **Szüts, Tamás, Dr. Dipl.-Ing. Chem.**
**Csalogány u. 53**
**H-1027 Budapest (HU)**

(74) Vertreter: **Patentanwälte Beetz sen. - Beetz jun.**
**Timpe - Siegfried - Schmitt-Fumian**
**Steinsdorfstrasse 10**
**D-8000 München 22 (DE)**

**Beschreibung**

Die Erfindung betrifft neue, pharmakologisch wirksame, in 7-Stellung substituierte Theophyllinderivate, Verfahren zu ihrer Herstellung sowie die neuen Verbindungen enthaltende Arzneimittel.

7-substituierte Theophyllinderivate besitzen die allgemeine Formel I

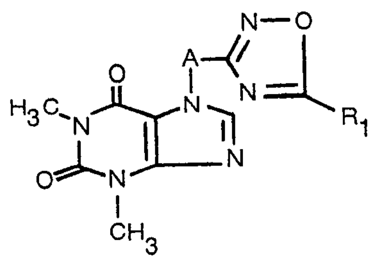

(I),

worin bedeuten:

A geradkettiges oder verzweigtes $C_{1-5}$-Alkylen oder

$$-CH_2-\underset{\displaystyle OH}{\underset{|}{CH}}-CH_2-$$

und

$R_1$ Hydroxy, geradkettiges oder verzweigtes $C_{1-10}$-Alkyl, -Halogenalkyl oder -Hydroxyalkyl, $C_{5-6}$-Cycloalkyl, Vinyl, 2-Ethoxyethyl, $C_{2-5}$-Carbonylalkyl, Benzyl, 2,2-Diphenylethyl, Theophyllin-7-ylmethyl, eine Aminoalkylgruppe der allgemeinen Formel 1,

$$-(CH_2)_n-\underset{\displaystyle R}{\underset{|}{CH}}-N\begin{matrix} R_2 \\ R_3 \end{matrix} \qquad (1),$$

oder

eine Phenylgruppe der allgemeinen Formel 2,

$$R_4R_5C_6H_3- \qquad (2),$$

wobei in Formel 1 bzw. 2 bedeuten:

R H oder Methyl,

$R_2$ und $R_3$ H oder geradkettiges oder verzweigtes $C_{1-4}$-Alkyl oder $R_2$ und $R_3$ zusammen mit dem N-Atom einen 5- oder 6-gliedrigen Heterocyclus, der ein gegebenenfalls durch Methyl substituiertes weiteres Stickstoffatom oder ein Sauerstoffatom enthalten kann,

n 0, 1, 2 oder 3 und

$R_4$ und $R_5$ unabhängig H, Chlor, Hydroxy, Methoxy, Ethoxy, Methyl oder Amino.

Wenn $R_1$ eine Hydroxylgruppe bedeutet, liegen die Verbindungen der allgemeinen Formel I je nach den Bedingungen in einer der tautomeren Formen Ia bzw. Ib

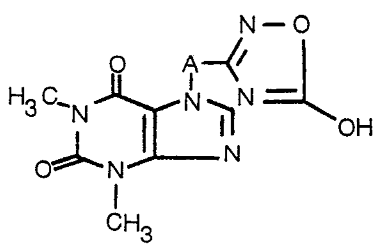

(Ia)

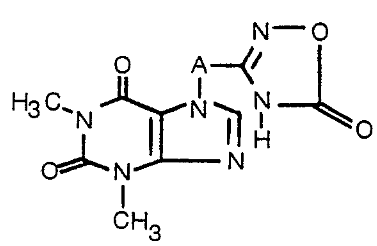

(Ib)

vor.

Zum Gegenstand der Erfindung gehören ferner die Salze der Verbindungen der allgemeinen Formel I. Vorzugsweise handelt es sich um die Säureadditionssalze, die aus den Verbindungen gewünschtenfalls gebildet werden. Zur Salzbildung können anorganische Säuren, wie Chlorwasserstoff, Bromwasserstoff, Schwefelsäure und Phosphorsäure, oder organische Säuren, wie Essigsäure, Bernsteinsäure, Glycolsäure, Milchsäure, Weinsäure, Citronensäure, Ascorbinsäure, Maleinsäure, Benzoesäure, Hydroxybenzoylbenzoesäure, Nicotinsäure oder Toluolsulfonsäure, verwendet werden. Zu den Salzen der Verbindungen der allgemeinen Formel I gehören ferner die mit niederen Alkylhalogeniden, Alkylsulfaten, Alkylphosphaten, vorzugsweise mit Methyljodid, Methylbromid oder Methylsulfat, gebildeten quaternären Ammoniumsalze.

Die Halogenalkylgruppen und Hydroxyalkylgruppen weisen 1 bis 10 C-Atome und bevorzugt 1 bis 5 C-Atome auf; die Halogenalkylgruppen sind vorzugsweise Chlormethyl-, Chlorethyl-, Chlorpropyl- oder Chlorbutylgruppen.

Wenn $R_2$ und $R_3$ mit dem Stickstoffatom gemeinsam einen 5- oder 6-gliedrigen heterocyclischen Ring bilden, handelt es sich bevorzugt um Piperidino-, Piperazino-, Pyrrolidino-, Morpholino- und N-Methylpiperazinogruppen.

Ein Teil der erfindungsgemäßen Verbindungen weist ein Chiralitätszentrum auf. Diese Verbindungen können sowohl als Racemate wie auch in optisch aktiver Form für pharmazeutische Zwecke eingesetzt werden. Unter den hier gebrauchten Bezeichnungen sind Racemate und optisch aktive Verbindungen gleichermaßen zu verstehen.

Als Mittel gegen Husten werden bekanntlich seit langem zumeist Codein und codeinartige Verbindungen pharmazeutisch eingesetzt. Diese Verbindungen wirken zentral durch Unterdrückung des Hustenreflexes. Da die zentrale Wirkung nicht spezifisch ist, haben Codein und die mit ihm verwandten Verbindungen zahlreiche unerwünschte Nebenwirkungen, unter anderem respirationshemmende Wirkung, weshalb ihre Anwendung in manchen Fällen, in erster Linie bei Asthmatikern kontraindiziert ist.

Es wurden daher neue Hustenmittel entwickelt, die nicht über das Zentralnervensystem wirken und deshalb die Atmung nicht behindern. Zu diesen neuen Hustenmitteln gehören auch bestimmte 1,2,4-Oxadiazole, deren pharmakologisch wichtigster Vertreter das Prenoxdiazin ist (vgl. die GB—A—1053825 sowie die DE—B—1063323-Libexin).

Es ist auch bekannt, daß Theophyllin und viele seiner Derivate eine vorteilhafte bronchienerweiternde, die Atmung erleichternde Wirkung haben. Diese Verbindungen spielen bei der Behandlung von Asthma eine wesentliche Rolle.

In der Arzneimittelforschung ist man in neuester Zeit bestrebt, durch Anwendung nicht respirationshemmender Hustenmittel die beiden günstigen Wirkungen, die hustenstillende sowie die bronchienerweiternde Wirkung, miteinander zu vereinigen.

Als Ergebnis dieser Bemühungen gibt es bis jetzt allerdings lediglich Hustenmittel, bei denen die Vereinigung der beiden wirkungen durch mechanisches Vermischen von zwei Verbindungen verschiedenen Typs bzw. durch Komplexbildung erreicht wurde. Derartige Lösungen sind z. B. in der BE—PS 874 773 bzw. in der entsprechenden US—PS 4 197 300 beschrieben.

Aus der FR—A—2 480 286 sind ferner 7-substituierte Theophyllinderivate bekannt, die einen Dioxolanring aufweisen, also cyclische Acetale darstellen, die sich vom Theophyllin-7-yl-acetaldehyd ableiten.

Diese Verbindungen weisen zwar bronchodilatorische und antitussive Wirksamkeit auf, besitzen jedoch zugleich die Eigenschaft, daß sie, ähnlich wie das zentraldämpfende Hustenmittel Codein, die Barbituratnarkose potenzieren (vgl. J. S. Franzone et al., Il Farmaco, Ed. Sc. 36, Bd. 3 (1981) 201—219).

Es wurde nun gefunden, daß die erfindungsgemäßen Theophyllinderivate der allgemeinen Formel I eine ausgezeichnete hustenstillende, entzündungshemmende und bronchienerweiternde Wirkung besitzen. In Abhängigkeit von den Substituenten ist die antitussive Wirkung zahlreicher Vertreter dieser Verbindungsgruppe überraschenderweise stärker als die des Prenoxdiazins und des Codeins, wobei diese Wirkung mit einer beträchtlichen, hinsichtlich der Wirkungsdauer die des Theophyllins übertreffenden bronchospasmolytischen und antiasthmatischen Wirkung verbunden ist. Es ist sowohl überraschend als auch aus therapeutischer Sicht günstig, daß die Toxizität der Verbindungen der allgemeinen Formel I nur einen Bruchteil der Toxizität von Prenoxdiazin bzw. Codein beträgt.

Aufgrund dieser außerordentlich günstigen pharmazeutischen Wirkungen können die Verbindungen der allgemeinen Formel I in der Humanmedizin, in erster Linie zur Behandlung von Erkrankungen der Atmungsorgane, zur Erweiterung der Bronchien, zur Vorbeugung und Behandlung von Asthma, als Hustenmittel sowie ferner als entzündungshemmende Mittel vorteilhaft eingesetzt werden.

Die antitussive Wirkung und die Toxizität einiger charakteristischer Vertreter der erfindungsgemäßen Verbindungen sowie zum Vergleich für die bekannten Mittel Prenoxdiazin und Codein sind in der Tabelle 1 zusammengefaßt. In Tabelle 1 entspricht der Wert Q der antitussiven Wirkung an Meerschweinchen bei durch 15 %igen Citronensäurespray ausgelöstem Husten, eine Stunde nach Verabreichung der Testverbindung (50 mg/kg i.m.), ausgedrückt in Prozent der Kontrolle.

TABELLE 1

| A | $R_1$ | Q, % | Toxizität (i.v.) | $LD_{50}$ Maus (p.o., mg/kg) |
|---|---|---|---|---|
| $CH_2$ | $CH_3$ | 54,31 | 416 | 1430 |
| $CH_2$ | $(CH_2)_2CH_3$ | 51,15 | 390 | 1400 |
| $CH_2$ | $(CH_2)_2N(C_2H_5)_2$ | 56,66 | 215 | 1405 |
| $CH_2$ | 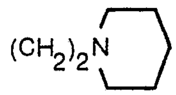 | 54,15 | 240 | 1550 |
| $CH_2$ | 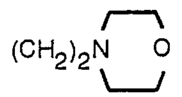 | 55,40 | 230 | 1530 |
| $CH_2$ | 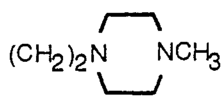 | 54,50 | 224 | 1670 |
| $(CH_2)_2$ | 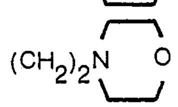 | 48,56 | 217 | 2600 |
| $(CH_2)_2$ | $(CH_2)_2NH$—i-$C_3H_7$ | 54,66 | 200 | 1750 |
| $(CH_2)_2$ | $(CH_2)_2N(C_2H_5)_2$ | 51,48 | 237 | 2250 |
| $(CH_2)_3$ | 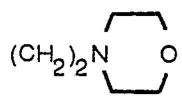 | 47,66 | 220 | 1900 |
| $CH_2CH(OH)CH_2$ | 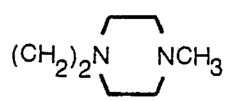 | 48,60 | 215 | 1800 |
| Prenoxdiazin | | 44,50 | 34 | 920 |
| Codein | | 50,00 | 54 | — |

Aus Tabelle 1 ist einesteils ersichtlich, daß die Verbindungen der allgemeinen Formel I in den meisten Fällen auch absolut eine bessere Wirkung haben, vor allem jedoch ihr therapeutischer Index viel günstiger ist, zum anderen, daß die Länge der Seitenkette A und die Variation des Substituenten $R_1$ weder bei der hustenstillenden Wirkung noch bei der Toxizität eine grundlegende Veränderung bewirken. Der größte Teil der erfindungsgemäßen Verbindungen kann in der Therapie sehr günstig eingesetzt werden. Das Verhältnis der i.v.- und der p.o.-Toxizität zeigt, daß die Verbindungen der allgemeinen Formel I im Verdauungssystem sehr gut absorbiert werden. Dies beweisen auch die für intraduodenale und für perorale Verabreichung aufgenommenen Dosis-Wirkungs-Kurven der antitussiven Wirkung.

Es muß auch betont werden, daß sich der größte Teil der Verbindungen der allgemeinen Formel I im Vergleich mit Prenoxdiazin in Wasser gut löst.

Diejenigen erfindungsgemäßen Verbindungen, bei denen $R_1$ Alkyl, Aralkyl oder Aryl bedeutet, verfügen über eine wertvolle, anhaltende antitussive und antiasthmatische Wirkung bzw. wirken bronchodilatorisch und entzündungshemmend. So verringert z. B. das 7-[(5-Methyl-1,2,4-oxadiazol-3-yl)-methyl]-theophyllin sowohl im Test in vitro am vom Kaninchen isolierten Tracheenstreifen wie auch im Test in vivo nach Konzett und Rössler (Arch. exp. Path. Pharmakol. 195 (1940) 71) am Meerschweinchen den durch Histamin ausgelösten Bronchialkrampf bedeutend und über längere Zeit als Theophyllinethylendiamin und weist auch in der Unterdrückung des durch Acetylcholin oder Serotonin provozierten Krampfes eine bedeutende Aktivität auf, was für die Anwendung gegen asthmatischen Husten von Bedeutung ist. Aufgrund der antitussiven Wirkung bzw. der an Mäusen und Ratten gemessenen Toxizität i.v. ist der therapeutische Index der genannten Verbindung etwa fünfmal so günstig wie der der Referenzsubstanz Codein und mehr als zehnmal so günstig wie der von Prenoxdiazin. Wie Untersuchungen an narkotisierten Katzen ergaben, erhöht die Verbindung das Atemvolumen und verringert gleichzeitig die Atemfrequenz, was für die Behandlung von Erkrankungen der Atmungsorgane besonders vorteilhaft ist. Die bedeutende therapeutische Wirkung der genannten Verbindungsgruppe besteht darin, daß die Verbindungen auch den

Ausbruch des durch verschmutzte Luft, in erster Linie durch Tabakrauch enthhaltende Luft provozierten chronischen Asthmas hemmen, wie an Ratten vorgenommene Langzeitversuche ergaben.

Eine andere Gruppe der Verbindungen der allgemeinen Formel I, bei denen $R_1$ eine Aminoalkylgruppe bedeutet, verfügen ebenfalls über antitussive und bronchodilatorische Wirkung, und daneben ist ihre entzündungshemmende Wirkung stark ausgeprägt. Die neben der aus Tabelle 1 hervorgehenden antitussiven Wirkung vorliegende entzündungshemmende Wirkung der Verbindung 7-[(5-[2-Diethylamino-ethan-1-yl]-1,2,4-oxadiazol-3-yl)-methyl]-theophyllin ist im Carrageenin-Ödemtest an der Rattenpfote etwa so stark wie die des Indomethacins, was in erster Linie für die Behandlung von zusammen mit Husten auftretenden Entzündungen der Atmungsorgane von Bedeutung ist.

Die Verbindungen der allgemeinen Formel I werden gemäß der Erfindung hergestellt durch

(A) Umsetzung von Amidoximen der allgemeinen Formel II

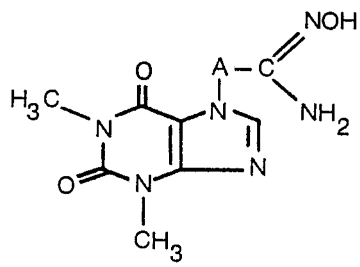

(II)

mit A wie in Formel I mit Säuren der allgemeinen Formel III

$$R_6\text{—COOH} \quad \text{(III)},$$

worin $R_6$ dasselbe wie $R_1$ in Formel I oder eine in $R_1$ überführbare Gruppe und vorzugsweise Vinyl, 2-Ethoxyethyl, $C_{3-4}$-Oxoalkyl, O-Tosylalkyl, O-Mesylalkyl oder Halogenalkyl und/oder acylierend wirkenden Derivaten der Säuren der allgemeinen Formel III unmittelbar zu Verbindungen der allgemeinen Formel Ic

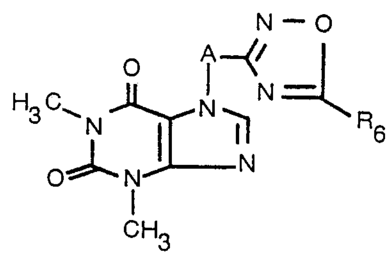

(Ic)

mit A und $R_6$ wie oben und erforderlichenfalls Umwandlung von $R_6$ in $R_1$ oder

(B) Umsetzung von Amidoximen der allgemeinen Formel II mit Säuren der allgemeinen Formel III oder acylierend wirkenden Derivaten der Säuren der allgemeinen Formel III zu acylierten Derivaten der allgemeinen Formel IV

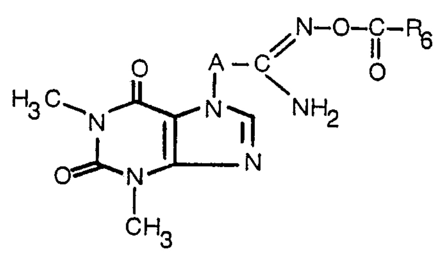

(IV)

mit A und $R_6$ wie oben und Cyclisierung der Verbindungen der allgemeinen Formel IV, gegebenenfalls nach isolierung, unter Wasserabspaltung und erforderlichenfalls Umwandlung von $R_6$ in $R_1$ oder

(C) Umsetzung von Verbindungen der allgemeinen Formel V

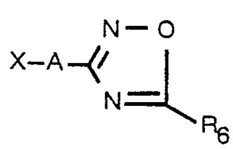

(V)

mit A und $R_6$ wie oben und X = Halogen oder eine Sulfonsäureestergruppe mit Theophyllin in Gegenwart eines basischen Katalysators oder mit einem mit einer Base gebildeten Salz des Theophyllins und erforderlichenfalls Umwandlung von $R_6$ in $R_1$ oder

(D) Umsetzung von Olefinen der allgemeinen Formel VI

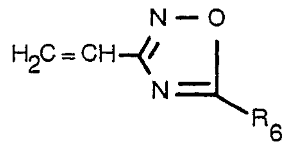

(VI)

mit $R_6$ wie oben in Gegenwart eines basischen Katalysators mit Theophyllin zu Verbindungen der allgemeinen Formel Id

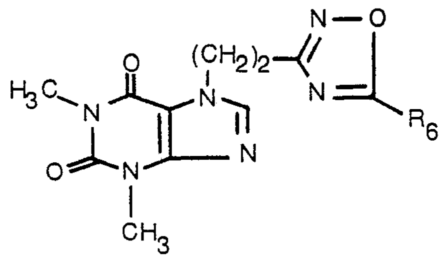

(Id)

mit $R_6$ wie oben und erforderlichenfalls Umwandlung von $R_6$ in $R_1$ oder

(E) Umsetzung von Epoxiden der allgemeinen Formel VII

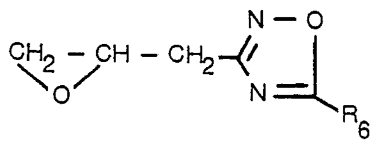

(VII)

mit $R_6$ wie oben mit Theophyllin zu einer Verbindung der Formel I mit

$$A = -CH_2-\underset{\displaystyle OH}{\underset{|}{CH}}-CH_2-$$

und erforderlichenfalls Umwandlung von $R_6$ in $R_1$.

Gemäß der Verfahrensvariante A setzt man das Amidoxim der allgemeinen Formel II zweckmäßig mit einem Ester der Formel $R_6COOR_7$, worin $R_7$ Alkyl, zweckmäßig Methyl oder Ethyl bedeutet, in Gegenwart einer Base, vorzugsweise eines Alkali- oder Erdalkalihydroxids, insbesondere von Kaliumhydroxid oder Natriumhydroxid, oder eines Alkalialkoxids, vorzugsweise von Natriummethylat oder Natriumethylat, und in Gegenwart eines polaren oder apolaren organischen Lösungs- und/oder Verdünnungsmittels unter Erwärmen, vorzugsweise bei 50 bis 150°C und besonders bevorzugt am Siedepunkt des verwendeten Lösungsmittels um. Im Falle von niedrigsiedenden Lösungsmitteln, z. B. Methanol, kann man auch unter Druck arbeiten, was eine den Siedepunkt des Lösungsmittels überschreitende Reaktionstemperatur ermöglicht.

Als polare Lösungsmittel werden bevorzugt Alkohole mit 1 bis 4 C-Atomen Carbonsäure-N-alkylamide, z.B. Dimethylformamid, und als unpolare Lösungsmittel aromatische Kohlenwasserstoffe, z.B. Benzol, Chlorbenzol, Toluol oder Xylol, verwendet. Das bei der Reaktion gebildete Wasser kann mit diesen Lösungsmitteln azeotrop abdestilliert werden.

Gemäß der Variante A) ist es ferner bevorzugt, das Amidoxim der allgemeinen Formel II mit einer Carbonsäure der allgemeinen Formel III und/oder deren Säureanhydrid unter Erwärmen in Gegenwart eines organischen Lösungsmittels umzusetzen. Als Lösungsmittel sind aromatische Kohlenwasserstoffe geeignet. Besonders vorteilhaft ist es, als Lösungsmittel einen überschuß des Acylierungsmittels zu verwenden. Die Acylierung und der Ringschluß werden bei Temperaturen von 50 bis 150°C, vorzugsweise bei 90 bis 110°C, vorgenommen.

Die Reaktionszeit hängt von den verwendeten Ausgangsstoffen und dem Lösungsmittel ab und kann zwischen 0,5 und 24 h variieren.

Gemäß der Verfahrensvariante B) nimmt man die Acylierungsreaktion zweckmäßig mit einem Säureanhydrid der allgemeinen Formel $(R_6CO)_2O$ oder einem Säurehalogenid der allgemeinen Formel $R_6COX$, worin X ein Halogenatom bedeutet, zweckmäßig mit Säurechloriden in Gegenwart eines organischen Lösungsmittels vor. Bevorzugte Lösungsmittel sind Aceton, Pyridin, Benzol, Toluol, Dimethylformamid und im Falle von Säureanhydriden überschüssiges Säureanhydrid, ferner Dialkylether mit 2 bis 4 C-Atomen im Alkylteil, Phenylalkylether, Dioxan, chlorierte Kohlenwasserstoffe mit 1 bis 4 C-Atomen, vorzugsweise Dichlormethan, Dichlorethan und Chloroform. Beim Acylieren mit einem Säurehalogenid ist es zweckmäßig, einen Säureakzeptor zuzusetzen. Als anorganische Säureakzeptoren

haben sich Alkali- und Erdalkalicarbonate, wie Natriumcarbonat, Kaliumcarbonat und Calciumcarbonat, ferner Alkalihydrogencarbonate, z.B. Natriumhydrogencarbonat, und als organische Säureakzeptoren tertiäre Amine, z. B. Pyridin und Triethylamin, als sehr vorteilhaft erwiesen, jedoch können auch das eingesetzte Amidoxim selbst sowie, falls eine basische Gruppe $R_6$ vorliegt, das entstehende acylierte Derivat der allgemeinen Formel IV als Säureakzeptoren dienen.

Der Ringschluß bei der Verfahrensvariante B) under Ausbildung des Oxadiazolrings wird entweder in Gegenwart eines polaren Lösungsmittels, vorzugsweise von organischen Säuren, Säureanhydriden, Pyridin oder Dimethylformamid, oder in Gegenwart eines unpolaren Lösungs- und/oder Verdünnungsmittels, wie eines aromatischen Lösungsmittels, besonders bevorzugt von Toluol, unter Erwärmen oder aber ohne Lösungsmittel pyrolytisch vorgenommen. Der Ringschluß der Verbindung der allgemeinen Formel IV kann auch in einem wasserhaltigen organischen Lösungsmittel bei einem pH-Wert zwischen 6 und 8, gegebenenfalls unter Erwärmen, vorgenommen werden. Als organische Lösungsmittel kommen mit Waser mischbare Lösungsmittel, z. B. Aceton oder Dioxan, oder Alkohole mit 1 bis 3 C-Atomen in Frage. Der Ringschluß wird, je nach der Löslichkeit der Verbindung der allgemeinen Formel IV und der Gruppe $R_6$, bei Temperaturen zwischen 20 und 100°C vorgenommen. In Wasser lösliche Verbindungen der allgemeinen Formel IV werden bevorzugt in einem wasserhaltigen organischen Lösungsmittel bei pH 6,6 bis 7,4 und einer Temperatur von 80 bis 100°C kondensiert. Zur optimalen pH-Einstellung kann das Vorliegen einer schwachen Base in katalytischer Menge vorteilhaft sein; als solche wird zweckmäßig das mit einer starken Base gebildete Salz der der Gruppe $R_6$ entsprechenden Carbonsäure, vorzugsweise das Natrium- oder Kaliumsalz, oder aber Natriumcarbonat, Kaliumcarbonat, Natriumhydrogencarbonat oder tertiäre Amine, besonders vorteilhaft Triethylamin, verwendet.

Die für die Verfahrensvarianten A) und B) erforderlichen Theophyllinyl-Alkylcarbonsäure-amidoxime können in an sich bekannter Weise, zweckmäßig durch Umsetzen der entsprechenden Theophyllinylalkylnitrile mit Hydroxylamin durch mehrstündiges Kochen in Methanol oder Ethanol, erhalten werden.

Bei der Verfahrensvariante C werden als Lösungs- und/oder Verdünnungsmittel vorzugsweise Dimethylformamid oder ein Alkohol, bevorzugt Ethanol oder Isopropanol, als Säureakzeptoren anorganische Basen, zweckmäßig ein Alkalihydroxid, vorzugsweise Kaliumhydroxid oder Natriumhydroxid, oder ein Alkalicarbonat, z.B. Natriumcarbonat oder Kaliumcarbonat, oder eine organische Base, vorzugsweise Pyridin oder Piperidin, verwendet. Die Ausgangsverbindung der allgemeinen Formel V wird bevorzugt mit einem Theophyllinalkalisalz, zweckmäßig Theophyllinnatrium oder -kalium, in Lösung oder Suspension, besonders zweckmäßig in Isopropanol, unter Erwärmen umgesetzt. Die Ausgangsverbindungen der allgemeinen Formel V werden in an sich bekannter Weise aus den entsprechenden 5-substituierten 3-(ω-Hydroxyalkyl)-1,2,4-oxadiazolen mit Thionylhalogeniden, vorzugsweise mit Thionylchlorid, oder mit Tosylchlorid bzw. Mesylchlorid (J. Chem. Research (M), 1979, 801) hergestellt.

Bei der Verfahrensvariante D setzt man einen basischen Katalysator, vorzugsweise ein quaternäres Ammoniumhydroxid, insbesondere Triton B, ein. Die für die Verfahrensvariante D als Ausgangsstoffe dienenden Olefine der allgemeinen Formel VI werden in literaturbekannter Weise (J. Chem. Research (M) 1979, 801) hergestellt.

Gemäß der Verfahrensvariante E wird ebenfalls bevorzugt ein basischer Katalysator, insbesondere Pyridin, eingesetzt; als Lösungsmittel wird bevorzugt Ethanol verwendet. Die als Ausgangsstoffe eingesetzten Epoxide der allgemeinen Formel VII werden in an sich bekannter Weise aus den entsprechenden, in 5-Stellung substituierten 3-(2,3-Dihydroxypropyl)-1,2,4-oxadiazolen durch Wasserentzug hergestellt.

Die erfindungsgemäßen Verbindungen der allgemeinen Forml Ic und diejenigen O-Acylamidoxime der allgemeinen Formel IV, bei denen $R_6$ Vinyl bedeutet, können aus den Amidoximen und den entsprechenden Acrylsäurederivaten, vorzugsweise Acrylsäurechlorid, gemäß den Verfahrensvarianten A und B, vorzugsweise in Aceton und in Gegenwart von Natriumcarbonat, hergestellt werden.

Die Verbindungen der allgemeinen Formel Ic, in der $R_6$ für 2-Ethoxyethyl steht, können aus den Amidoximen der allgemeinen Formel II durch Umsetzen mit Ethylacrylat in ethanolischem Medium und Gegenwart von Natriumethylat bei erhöhter Temperatur hergestellt werden.

Die Verbindungen der allgemeinen Formeln Ic und IV, in denen $R_6$ für Halogenalkyl steht, können durch Umsetzen der entsprechenden halogenalkancarbonsäurechloride mit den Amidoximen der allgemeinen Formel II hergestellt werden. Die Acylierung wird zweckmäßig unter Kühlung in Aceton in Gegenwart eines Alkalicarbonats vorgenommen. Der Ringschluß der acylierten Verbindung zu der Verbindung Ic erfolgt zweckmäßig in Toluol oder durch Pyrolyse.

Die Verbindungen der allgemeinen Formel Ic und O-Acylamidoxime der allgemeinen Formel IV, bei denen $R_6$ für Vinyl, 2-Ethoxyethyl, $C_{1-4}$-Halogenalkyl, O-Mesylalkyl oder O-Tosylalkyl steht, können mit Aminen der Formel $NHR_2R_3$ zu Verbindungen der allgemeinen Formel I umgesetzt werden, bei denen $R_1$ eine Gruppe $-(CH_2)_2NR_2R_3$ bzw. $-(CH_2)_nNR_2R_3$ bedeutet.

Die Aminierung wird zweckmäßig mit einem Aminüberschuß von 5 bis 100% in einem organischen Lösungsmittel, zweckmäßig in Toluol, unter Erwärmen auf 50 bis 150°C vorgenommen. Sehr zweckmäßig ist es, die Acylierung, die Aminierung und den Ringschluß ohne Isolierung der Zwischenprodukte vorzunehmen.

Die Theophyllinderivate der allgemeinen Formel Ic, bei denen $R_6$ für eine Oxoalkylgruppe steht, werden durch Acylierung der Amidoxime der allgemeinen Formel II mit den entsprechenden Oxoalkancarbonsäurederivaten und Ringschluß erhalten. Die Oxoalkylgruppe kann auch mit Aminen der allgemeinen Formel $R_8NH_2$, worin $R_8$ mit Ausnahme von Wasserstoff alle Bedeutungen von $R_2$ haben kann, durch reduktive Kondensation in sehr vorteilhafter Weise zu der entsprechenden sekundären Aminoalkylgruppe umgesetzt werden. Da sich der 1,2,4-Oxadiazolring bei katalytischer Hydrierung öffnen würde, wird die reduktive Kondensation zweckmäßig durch Reduktion der Azomethinbindung mit Natriumtetrahydroborat in einem Lösungsmittelmedium vorgenommen.

Diejenigen Verbindungen der allgemeinen Formel Ic, bei denen $R_6$ Aminoalkyl $-(CH_2)_nNH_2$ bedeutet, worin n 1, 2, 3 oder 4 ist, werden mit Alkylhalogeniden, Alkylsulfaten oder Alkylphosphaten zu Verbindungen der allgemeinen Formel I alkyliert, bei denen $R_1$ $-(CH_2)_n(R_2)_2$ oder $-(CH_2)_n-N(R_2)_3Q^-$ bedeutet, wobei $Q^-$ das dem Alkylierungsmittel entsprechende Anion ist. Die Methylierung der primären Aminogruppe wird zweckmäßig in Ameisensäure mit Formaldehyd vorgenommen.

Die erfindungsgemäßen Verbindungen der allgemeinen Formel I werden zweckmäßig in Form von Arzneimittelpräparaten eingesetzt, die bevorzugt eine einmalige Dosis enthalten. Zur Formulierung werden die in der Pharmazie üblichen anorganischen und organischen Träger-, Streck- und Hilfsmittel verwendet. Die Arzneimittel sind zur enteralen oder parenteralen Verabreichung geeignet. Bevorzugt sind als Formulierungsformen Sirupe, Tabletten, Kapseln und Suppositorien; neben den Wirkstoffen enthalten die Formulierungen Streckmittel, z. B. Lactose, Dextrose, Saccharose, Mannit, Sorbit, Cellulose und ihre Derivate, Glycerin, Gleitstoffe, z. B. Kieselerde, Talkum, Stearinsäure und deren Salze und Polyethylenglycole, Bindemittel, wie Silicate, Stärke und ihre Derivate, Gelatine, Methylcellulose, Füllstoffe, Schäummittel, färbende Substanzen sowie Geschmacksmittel. Die erfindungsgemäßen Verbindungen können auch in zur parenteralen Verabreichung geeigneter Weise als Injektions- oder Infusionslösungen formuliert sein. Die Arzneimittelpräparate, die erforderlichenfalls weitere pharmazeutisch wirksame Stoffe enthalten, werden in an sich bekannter Weise mit Hilfe der üblichen Misch-, Granulierungs-, Dragierungs-, Lösungs- und Lyophilisierungsverfahren hergestellt. Sie enthalten etwa 0,2 bis 100% und vorzugsweise etwa 1 bis 50% Wirkstoff. Die Dosierung hängt von verschiedenen Faktoren, z. B. der Applikationsart sowie Alter und Zustand der Patienten, ab. Bei oraler Verabreichung beträgt die tägliche Dosis etwa 0,1 bis 2,0 g bei einem Körpergewicht von etwa 70 kg.

Die Erfindung wird anhand der folgenden Beispiele näher erläutert.

## Beispiel 1

a) 25,2 g 2-(Theophyllin-7-yl)-acetamidoxim werden in 250 ml Essigsäureanhydrid unter Erwärmen aufgelöst. Man läßt die Lösung über Nacht stehen und versetzt am nächsten Tag mit 250 ml Diethylether. Es werden 27,6 g 2-(Theophyllin-7-yl)-O-acetylacetamidoxim erhalten (Ausbeute 94%); F. 201°C (aus Ethanol).

b) 25,2 g 2-(Theophyllin-7-yl)-acetamidoxim werden in einem Gemisch aus 400 ml wasserfreiem Aceton und 10,2 g Triethylamin unter Rühren mit 78,5 g Acetylchlorid umgesetzt. Es werden 23,1 g 2-(Theophyllin-7-yl)-O-acetylacetamidoxim erhalten (Ausbeute 78%); F. 201°C.

c) 2,94 g 2-(Theophyllin-7-yl)-O-acetylacetamidoxim werden in 20 ml Pyridin 2 h lang auf dem Wasserbad erwärmt. Dann wird das Lösungsmittel unter vermindertem Druck abdestilliert. Der Rückstand wird aus Wasser kristallisiert. Es werden 2,58 g 7-[(5-Methyl-1,2,4-oxadiazol-3-yl)-methyl]-theophyllin erhalten (Ausbeute 93%); F. 135 bis 136°C.

Der Ringschluß kann statt in Pyridin auch in Essigsäureanhydrid, Essigsäure oder einem Gemisch dieser beiden Verbindungen vorgenommen werden.

d) 15,12 g 2-(Theophyllin-7-yl)-acetamidoxim werden in einem Gemisch aus 120 ml Essigsäure und 8 ml Essigsäureanhydrid 2 h auf dem Wasserbad erwärmt. Nach dem Abdestillieren des Lösungsmittel erhält man 16,06 g 7-[(5-Methyl-1,2,4-oxadiazol-3-yl)-methyl]-theophyllin (Ausbeute 91%); F. 135°C (aus Methanol).

e) Ein Gemisch aus 5,04 g 2-(Theophyllin-7-yl)-acetamidoxim, 2,16 g Natriummethylat, 10 ml Ethylacetat und 200 ml Toluol wird unter Rühren unter einem Wasserabscheideaufsatz 20 h lang gekocht. Während dieser Zeit werden insgesamt noch 10 ml Ethylacetat in fünf Portionen zu dem Gemisch zugegeben. Nach Abdampfen des Lösungsmittels und Kristallisieren aus Wasser erhält man 4,1 g 7-[(5-Methyl-1,2,4-oxadiazol-3-yl)-methyl]-theophyllin (Ausbeute 74%); F. 135 bis 136°C.

f) Ein Gemisch aus 5,04 g 2-(Theophyllin-7-yl)-acetamidoxim, 2,16 g Natriummethylat und 10 ml Ethylacetat wird in 150 ml Methanol unter Druck und ständigem Schütteln 8 h lang auf 100°C gehalten. Nach dem Aufarbeiten erhält man 4,8 g 7-[(5-Methyl-1,2,4-oxadiazol-3-yl)-methyl]-theophyllin (Ausbeute 87%), F. 135 bis 136°C.

g) Ein Gemisch aus 20,2 g Theophyllinnatrium, 300 ml Isopropanol und 13,2 g 3-Chlormethyl-5-methyl-1,2,4-oxadiazol wird unter Rühren 10 h lang gekocht. Nach dem Aufarbeiten erhält man 20,5 g 7-[(5-Methyl-1,2,4-oxadiazol-3-yl)-methyl]-theophyllin (Ausbeute 74,2%).

h) 2,94 g 2-(Theophyllin-7-yl)-O-acetylacetamidoxim werden in 20 ml Essigsäure 2 h lang gekocht. Es werden 2,62 g 7-[(5-Methyl-1,2,4-oxadiazol-3-yl)-methyl]-theophyllin erhalten (Ausbeute 94%); F. 134 bis 135°C.

i) 29,42 g 2-(Theophyllin-7-yl)-O-acetylamidoxim werden in 400 ml Wasser von 97 bis 99°C aufgelöst, worauf der pH-Wert der Lösung mit Triethylamin auf 7 eingestellt wird. Die Lösung wird 5,5 h lang auf der genannten Temperatur gehalten und dann mit Salzsäure auf pH 1 eingestellt. Nach Extraktion mit 4 × 50 ml

Dichlorethan, Abdestillieren des Lösungsmittels und Umkristallisieren des Rückstands aus Wasser werden 24,5 g 7-[(5-Methyl-1,2,4-oxadiazol-3-yl)-methyl]-theophyllin erhalten; F. 135 bis 136°C.

j) Das in den vorhergehenden Beispielen verwendete Amidoxim wird folgendermaßen hergestellt:

10,96 g 7-Cyanomethyltheophyllin, 7,64 g Hydroxylaminhydrochlorid, 5,98 g Natriummethylat und 50 ml Methanol werden auf dem Wasserbad fünf h lang gekocht. Es werden 9,85 g 2-(Theophyllin-7-yl)-acetamidoxim erhalten (Ausbeute 78%; F. 224°C (aus wäßrigem Ethanol).

k) Das als Ausgangsstoff verwendete O-Acetylamidoxim wird folgendermaßen hergestellt:

Ein Gemisch aus 25,42 g 2-(Theophyllin-7-yl)-acetamidoxim, 100 ml Dichlorethan und 10,8 g Essigsäureanhydrid wird 3 h lang bei 50 bis 55°C gerührt. Nach der Aufarbeitung werden 28,8 g 2-(Theophyllin-7-yl)-O-acetyl-acetamidoxim erhalten (F. 197 bis 198°C), das sofort weiterverarbeitet werden kann.

Beispiel 2

25,2 g 2-(Theophyllin-7-yl)-acetamidoxim werden in 400 ml wasserfreiem Aceton in Gegenwart von 8,6 g Natriumhydrogencarbonat mit einer Lösung von 11,3 g Chloracetylchlorid in 40 ml Aceton acyliert. Es werden 27 g 2-(Theophyllin-7-yl)-O-chloracetylacetamidoxim erhalten (Ausbeute 83%). Das Produkt wird im Vakuum (133 Pa) bei 105°C bis zur Gewichtskonstanz getrocknet (etwa 20 bis 60 min). Nach Umkristallisieren aus Methanol werden 19,1 g 7-[(5-Chlormethyl-1,2,4-oxadiazol-3-yl)-methyl]-theophyllin erhalten (Ausbeute 62%); F. 146 bis 148°C.

Beispiel 3

a) Ein Gemisch aus 5,04 g 2-(Theophyllin-7-yl)-acetamidoxim, 2,16 g Natriummethylat, 6,37 g Diethylaminoessigsäureethylester und 100 ml Toluol wird gekocht. Das kochende Gemisch wird eingedampft und der Eindampfrückstand aus Cyclohexan umkristallisiert. Es werden 5,4 g 7-[(5-Diethylaminomethyl-1,2,4-oxadiazol)-methyl]-theophyllin erhalten (Ausbeute 82%); F. 68 bis 70°C. Das Hydrochlorid schmilzt bei 206 bis 210°C.

b) Ein Gemisch aus 9,0 g 7-[(5-Chlormethyl-1,2,4-oxadiazol)-methyl]-theophyllin, 6 ml Diethylamin und 50 ml Toluol wird 8 h auf dem Wasserbad gerührt und dann unter vermindertem Druck eingedampft. Der Rückstand wird mit Wasser ausgewaschen und dann in 50 ml Ethanol heiß gelöst. Die Lösung wird mit Aktivkohle geklärt und mit salzsaurem Ethanol versetzt. Es werden 8,3 g 7-[(5-Diethylaminomethyl-1,2,4-oxadiazol-3-yl)-methyl]-theophyllin-hydrochlorid erhalten; F. 207 bis 210°C.

c) Zu einer Lösung von 5,04 g 2-(Theophyllin-7-yl)-acetamidoxim in 40 ml wasserfreiem Pyridin werden unter Rühren und Kühlen (max. 20°C) 3,0 g Diethylaminoacetylchlorid zugetropft. Das Reaktionsgemisch wird 1 h lang bei Raumtemperatur und dann 2 h lang auf dem Wasserbad gerührt. Das Gemisch wird danach zur Trockne eingedampft; aus dem Rückstand wird in Ethanol das Hydrochlorid hergestellt. Es werden 6,1 g 7-[(5-Diethylaminomethyl-1,2,4-oxadiazol-3-yl)-methyl]-theophyllin-hydrochlorid erhalten; F. 208 bis 210°C.

d) Zu einer Suspension von 3,27 g des gemäß Beispiel 2 hergestellten 2-(Theophyllin-7-yl)-O-chloracetylacetamidoxims in 20 ml Toluol werden 3 ml Diethylamin getropft. Das Gemischh wird 8 h lang gekocht. Das mit salzsaurem Ethanol gebildete 7-[(5-Diethylaminomethyl-1,2,4-oxadiazol-3-yl)-methyl]-theophyllin-hydrochlorid wird in einer Menge von 2,6 g erhalten (Ausbeute 71%); F. 207 bis 209°C.

e) Ein Gemisch aus 5,04 g 2-(Theophyllin-7-yl)-acetamidoxim, 2,16 g Natriummethylat, 6,37 g Diethylaminoessigsäureethylester und 80 ml Methanol wird bei 100°C in einem geschlossenen System unter Druck 10 h lang gerührt. Nach dem Aufarbeiten werden 5,2 g 7-[(5-Diethylaminomethyl-1,2,4-oxadiazol-3-yl)-methyl]-theophyllin erhalten (Ausbeute 80%); F. 69 bis 70°C (aus Cyclohexan).

f) 30,9 g 7-[(5-Chlormethyl-1,2,4-oxadiazol-3-yl)-methyl]-theophyllin und 18,5 g Phthalimidkalium werden in 300 ml Dimethylformamid unter ständigem Rühren 6 h lang gekocht. Das Dimethylformamid wird unter vermindertem Druck abdestilliert, der Rückstand mit 300 ml Ethanol verrieben, mit 5,2 g Hydrazinhydrat versetzt und 2 h auf dem Wasserbad erwärmt. Dann wird das Gemisch mit wäßriger Salzsäure stark angesäuert, aufgekocht und kochend filtriert. Das Filtrat wird eingedampft. Durch Kristallieren aus Methanol erhält man 24,7 g 7-[(5-Aminomethyl-1,2,4-oxadiazol-3-yl)-methyl]-theophyllin-hydrochlorid (Ausbeute 74,2%); F. 204 bis 207°C.

g) 3,34 g 7-[(5-Aminomethyl-1,2,4-oxadiazol-3-yl)-methyl]-theophyllin-hydrochlorid, 0,8 g Natriumformiat und 30 ml 90-%ige Ameisensäure werden auf dem Wasserbad erwärmt, bis alles geschmolzen ist. Nach dem Abkühlen werden 4,5 ml 30-%ige Formaldehydlösung zugegeben.

Das Gemisch wird 8 h auf dem Wasserbad erwärmt und dann zur Trockne eingedampft; der Rückstand wird mit 10 ml 10-%iger Natronlauge verrieben. Die organische Substanz wird mit Chloroform extrahiert. Mit salzsaurem Ethanol werden 1,95 g 7-[(5-Dimethylaminomethyl-1,2,4-oxadiazol-3-yl)-methyl]-theophyllin-hydrochlorid erhalten (Ausbeute 55%); F. 211 bis 213°C.

h) 3,34 g 7-[(5-Aminomethyl-1,2,4-oxadiazol-3-yl)-methyl]-theophyllin-hydrochlorid und 4,54 g Kaliumcarbonat werden in 80 ml Dimethylformamid bei Raumtemperatur mit 2,2 g Ethylbromid versetzt und 7 h lang gerührt. Das Lösungsmittel wird dann unter vermindertem Druck abdestilliert. Mit salzsaurem Ethanol werden 2,2 g 7-[(5-Diethylaminomethyl-1,2,4-oxadiazol-3-yl)-methyl]-theophyllin-hydrochlorid erhalten (Ausbeute 62%); F. 207 bis 208°C (Ethanol).

Beispiel 4

a) Ein Gemisch aus 25,2 g 2-(Theophyllin-7-yl)-acetamidoxim, 200 ml Toluol, 6.8 g Natriummethylat und 34,6 g β-Diethylaminopropionsäureethylester wird in einem mit einem Wasserabscheider versehenen Kolben unter ständigem Rürhen 4 h lang gekocht. Nach dem Filtrieren wird mit 11,6 g Maleinsäure das Salz gebildet. Es werden 40,5 g 7-[(5-[2-Diethylamino-ethan-1-yl]-1,2,4-oxadiazol-3-yl)-methyl]-theophyllin-maleinat erhalten (Ausbeute 85%); F. 127 bis 128°C.

b) Ein Gemisch aus 5,04 g 2-(Theophyllin-7-yl)-acetamidoxim, 2,16 g Natriummethylat, 6,4 g Diethylaminopropionsäureethylester und 100 ml Toluol wird gemäß Stufe a) umgesetzt. Es werden 6,3 g 7-[(5-[2-Diethylaminoethan-1-yl]-1,2,4-oxadiazol-3-yl)-methyl]-theophyllin erhalten (Ausbeute 87%); F. 69 bis 70°C (Cyclohexan).

c) Zu einem Gemisch aus 25,2 g 2-(Theophyllin-7-yl)-acetamidoxim, 400 ml Aceton und 8,6 g Natriumhydrogencarbonat wird eine Lösung von 9,1 g Acrylsäurechlorid in 40 ml Aceton gegeben. Es werden 28,1 g 2-(Theophyllin-7-yl)-O-acroylacetamidoxim erhalten; F. 160 bis 165°C (Methanol).

d) 6,12 g rohes 2-(Theophyllin-7-yl)-O-acroylacetamidoxim werden mit 25 ml Diethylamin auf einem 110°C warmen Bad 6 h lang erwärmt. Die base wird unter vermindertem Druck abdestilliert, der Rückstand wird aus Cyclohexan kristalliert. Es werden 5,8 g 7-[(5-[2-Diethylaminoethan-1-yl]-1,2,4-oxadiazol-3-yl)-methyl]-theophyllin erhalten (Ausbeute 80%); F. 78 bis 80°C.

e) Ein Gemisch aus 12,6 g 2-(Theophyllin-7-yl)-acetamidoxim, 10 g Ethylacrylat, 5,2 ml Diethylamin, 3,4 g Natriumethylat und 200 ml Ethanol wird bei 100°C unter Druck und ständigem Rühren 15 h lang gekocht. Dann wird mit 5,8 g Maleinsäure das Salz gebildet. Es werden 14,5 g 7-[(5-[2-Diethylaminoethan-1-yl)-1,2,4-oxadiazol-3-yl)-methyl]-theophyllin-maleinat erhalten (Ausbeute 61%); F. 126 bis 128°C.

f) Ein Gemisch aus 12,6 g 2-(Theophyllin-7-yl)-acetamidoxim, 10 g Ethylacrylat, 280 ml Ethanol und 3,4 g Natriumethylat wird unter Rühhren 15 h lang gekocht. Es werden 16,0 g rohes 7-[(5-[2-Ethoxymethyl]-1,2,4-oxadiazol-3-yl)-methyl]-theophyllin erhalten (Ausbeute 95%). Das Produkt wird in 20 ml Diethylamin unter Rühren 8 g lang in einem Ölbad auf 110°C gehalten. Dann wird unter vermindertem Druck eingedampft, der Rückstand durch Verreiben mit Wasser ausgewaschen und in siedendem Ethanol mit 5,8 g Maleinsäure das Salz gebildet. Es werden 16,2 g 7-[(5-[2-Diethylaminoethan-1-yl]-1,2,4-oxadiazol-3-yl)-methyl]-theophyllin-maleinat erhalten (Ausbeute 68%) F. 125 bis 128°C.

g) Ein Gemisch aus 5,04 g 2-(Theophyllin-7-yl)-acetamidoxim, 4,8 g Hydracrylsäureethylester, 2,16 g Natriumethylat und 100 ml Methanol wird unter Rühren und Druck 12 h lang auf 100°C gehalten. Nach dem Abdestillieren des Lösungsmittels wird der Rückstand aus Wasser kirstallisiert. Es werden 5,3 g 7-[(5-[2-Hydroxyethyl]-1,2,4-]oxadiazol-3-yl)-methyl]-theophyllin erhalten (Ausbeute 87%); F. 145°C. Die rohe Verbindung wird in einem Gemisch aus 20 ml Thionylchlorid und 20 ml Benzol 2 h lang gekocht. Nach dem Abdestillieren des Lösungsmittels bleibt 7-[(5-[2-Chlorethyl]-1,2,4-oxadiazol-3-yl)-methyl]-theophyllin zurück, das nach Zusatz von 50 ml Dimethylformamid, 6 ml Diethylamin und 5 g Kaliumcarbonat unter intensivem Rühren 10 h lang auf 100°C gehalten wird. Es werden 4,4 g 7-[(5-[2-Diethylaminoethyl]-1,2,4-oxadiazol-3-yl)-methyl]-theophyllin erhalten (Ausbeute 61%); F. 65 bis 68°C (Cyclohexan).

Beispiel 5

Ein Gemisch aus 5,04 g 2-(Theophyllin-7-yl)-acetamidoxim, 5,76 g Laevulinsäureethylester, 1,12 g Kaliumhydroxid und 100 ml Toluol wird in einem mit einem Wasserabscheider versehenen Kolben unter Rühren 1,8 h lang gekocht. Es werden 5,3 g 7-[(5-[Butan-3-on-1-yl]-1,2,4-oxadiazol-3-yl)-methyl]-theophyllin erhalten (Ausbeute 83%); F. 135 bis 140°C (Ethanol).

Beispiel 6

3,32 g des gemäß dem vorhergehenden Beispiel hergestellten 7-[(5-[Butan-3-on-1-yl]-1,2,4-oxadiazol-3-yl)-methyl]-theophyllins werden in 80 ml Benzol mit 6 ml Isopropylamin unter Druck auf 80°C erwärmt und 4 h lang auf dieser Temperatur gehalten. Nach dem Eindampfen wird der Rückstand in 80 ml Methanol gelöst und mit 0,8 g Natriumtetrahydroborat reduziert. Es werden 2,0 g 7-[(5-[3-Isopropylaminobutan-1-yl]-1,2,4-oxadiazol-3-yl)-methyl]-theophyllin erhalten (Ausbeute 53%); F. 55 bis 61°C.

Beispiel 7

Ein Gemisch aus 25,2 g 2-(Theophyllin-7-yl)-acetamidoxim, 200 ml Toluol, 6,8 g Natriummethylat und 37,4 g γ-Diethylaminobuttersäureethylester wird gemäß Beispiel 4a) umgesetzt. Es werden 38 g 7-[(5-[3-Diethylaminopropan-1-yl]-1,2,4-oxadiazol-3-yl)-methyl]-theophyllin-maleinat erhalten (Ausbeute 77%, F. 119 bis 121°C.

Beispiel 8

Ein Gemisch aus 25,2 g 2-(Theophyllin-7-yl)-acetamidoxim, 200 ml Toluol, 6,8 g Natriummethylat und 40,2 g δ-Diethylaminovaleriansäureethylester wird auf die in Beispiel 4a) beschriebene Weise umgesetzt. Man erhält 40,9 g 7-[(5-[4-Diethylamino-butan-1-yl]-1,2,4-oxadiazol-3-yl]-1,2,4-oxadiazol-3-yl)-methyl]-theophyllin-maleinat (Ausbeute 81%); F. 130 bis 132°C.

Beispiel 9

a) 13,3 g β-(Theophyllin-7-yl)-propionsäureamidoxim werden in 40 ml Essigsäureanhydrid gelöst und 1 h lang gekocht. Es werden 5,1 g 7-[2-(5-Methyl-1,2,4-oxadiazol-3-yl)-ethan-1-yl]-theophyllin erhalten (Ausbeute 88%); F. 115 bis 116°C (Ethylacetat).

b) Ein Gemisch aus 2,6 g β-(Theophyllin-7-yl)-propionsäureamidoxim, 0,68 g Natriumethylat, 10 ml Ethylacetat und 30 ml Ethanol wird unter Druck auf 110°C erwärmt und 12 h auf dieser Temperatur gehalten. Es werden 2,2 g 7-[2-(5-Methyl-1,2,4-oxadiazol-3-yl)-ethan-1-yl]-theophyllin erhalten (Ausbeute 76%); F. 113 bis 115°C (Ethanol).

c) Ein Gemisch aus 2,2 g 3-Vinyl-5-methyl-1,2,4-oxadiazol, 30 ml Dimethylformamid, 0,2 ml Triton B als Katalysator und 3,6 g Theophyllin wird 1 h lang gekocht. Das Lösungsmittel wird abdestilliert und der Rückstand aus Ethanol kristallisiert. Es werden 4,2 g 7-[2-(5-Methyl-1,2,4-oxadiazol-3-yl)-ethan-1-yl]-theophyllin erhalten (Ausbeute 72%); F. 112 bis 114°C.

d) Ein Gemisch aus 2,92 g 3-(2-Chlorethyl)-5-methyl-1,2,4-oxadiazol, 50 ml Isopropanol und 4,0 g Theophyllinnatrium wird 8 h lang gekocht. Es werden 5,3 g 7-[2-(5-Methyl-1,2,4-oxadiazol-3-yl)-ethan-1-yl]-theophyllin erhalten (Ausbeute 91%); F. 113 bis 114°C (Wasser).

Beispiel 10

a) Eine Lösung von 39,19 g metallischem Natrium in 500 ml Methanol wird zu einer Lösung von 118,4 g Hydroxylaminhydrochlorid in 1 l kochendem Methanol gegeben. Nach dem Filtrieren wird das Filtrat zu 112,6 g 7-(2-Hydroxy-3-cyanopropyl-1-yl)-theophyllin (hergestellt gemäß der DE—B—1 064 066) gegeben. Das Gemisch wird 30 h lang erwärmt. Es werden 125,0 g 3-(Theophyllin-7-yl)-2-hydroxypropionsäureamidoxim erhalten (Ausbeute 93%); F. 200 bis 202°C.

b) 6,28 g des gemäß a) hergestellten Amidoxims werden zusammen mit 1,38 g Natriumethylat, 30 ml Ethylacetat und 100 ml Ethanol unter Rühren 12 h lang gekocht. Es werden 5,7 g 7-[2-Hydroxy-3-(5-methyl-1,2,4-oxadiazol-3-yl)-propan-1-yl]-theophyllin erhalten (Ausbeute 89%); F. 148 bis 150°C (Ethanol).

c) 1,4 g 3-(2,3-Epoxypropyl)-5-methyl-1,2,4-oxadiazol und 1,8 g Theophyllin werden in einem Gemisch aus 20 ml Isopropanol und 0,1 ml Pyridin unter Rühren 8 h lang gekocht. Es werden 2,75 g 7-[2-Hydroxy-3-(5-methyl-1,2,4-oxadiazol-3-yl)-propan-1-yl]-theophyllin erhalten (Ausbeute 86%).

Beispiel 11

Ein Gemisch aus 12,56 g des gemäß Beispiel 10a) hergestellten Amidoxims, 14,80 g β-Piperidinopropionsäureethylester, 2,16 g Natriummethylat und 200 ml Toluol wird 5 h lang gekocht. Nach dem Eindampfen wird der Rückstand mit einem Gemisch aus Benzol und Ether im Verhältnis 1:1 behandelt. 11,7 g 7-[2-Hydroxy-3-(5-[2-piperidinoethan-1-yl]-1,2,4-diazol-3-yl)-propan-1-y)]-theophyllin erhalten (Ausbeute 71%); F. 110 bis 111°C. F. des Maleinats 179 bis 180°C.

Beispiel 12

a) Ein Gemisch aus 57,9 g 7-(3-Chlorpropan-1-yl)-theophyllin, 12,29 g Natrium cyanid, 2,0 g Natriumjodid und 400 ml Dimethylformamid wird bei 90°C 3 h lang gerührt. 49,1 g (88%) γ-(Theophyllin-7-yl)-butyronitril werden erhalten, das bei 146 bis 150°C schmilzt.

b) Aus dem gemäß Beispiel 12a) hergestellten Nitril wird ähnlich wie in Beispiel 1j) beschrieben mit Hydroxylamin in 94-%iger Ausbeute γ-(Theophyllin-7-yl)-buttersäureamidoxim erhalten, das sich bei 190°C zu verfärben beginnt.

c) Ein Gemisch aus 7,29 g γ-(Theophyllin-7-yl)-buttersäureamidoxim, 9,01 g β-Diethylaminopropionsäureethylester, 1,4 g Natriummethylat und 80 ml Toluol wird 2 h lang gekocht. Mit Maleinsäure werden 10,45 g 7-[3-(5-[2-Diethylaminoethan-1-yl]-1,2,4-oxadiazol-3-yl)-propan-1-yl]-theophyllin-maleinat erhalten (Ausbeute 80%); F. 134 bis 136°C.

Beispiel 13

a) Ein Gemisch aus 3,85 g 2-(4-Chlorbutan-1-yl-oxy)-pyran, 4,04 g Theophyllinnatrium, 0,01 g Natriumjodid und 15 ml Dimethylformamid wird unter Rühren 2 h lang auf 110°C gehalten. Das Lösungsmittel wird dann unter vermindertem Druck abdestilliert, der Rückstand mit Wasser verrieben, mit Chloroform extrahiert und aus dem Extrakt das Chloroform abdestilliert. Das zurückbleibende zähe Öl wird in einem Gemisch aus 30 ml 96-%igem Ethanol und 2 g DOWEX 50 W unter Rühren 8 h lang gekocht. Die Lösung wird filtriert und das Filtrat eingedampft. Der Rückstand wird aus Ethylacetat umkristallisiert. Es werden 4,2 g 4-(Theophyllin-7-yl)-butan-1-ol erhalten (Ausbeute 85%).

b) Ein Gemisch aus 2,52 g des gemäß Stufe a) erhaltenen Theophyllinylbutanols, 30 ml Benzol und 0,9 ml Thionylchlorid wird 1 h lang gekocht., Das nach dem Abdestillieren des Lösungsmittels zurückbleibende Öl kristallisiert beim Stehen. Es werden 2,61 g 1-Chlor-4-(theophyllin-7-yl)-butan erhalten (Ausbeute 96%); F. 91 bis 93°C.

c) Ein Gemisch aus 2,7 g des gemäß Stufe b) hergestellten Chlortheophyllinylbutans, 0,51 g Natriumcyanid, 0,01 g Natriumjodid und 10 ml Dimethylformamid wird 5 h lang auf 95°C gehalten. Es werden 1,9 g δ-(Theophyllin-7-yl)-valeronitril erhalten (Ausbeute 73%); F. 118 bis 120°C (Ethylacetat).

d) Auf die in Beispiel 12b) beschriebene Weise wird aus dem Nitril mit Hydroxylamin in 82-%iger Ausbeute δ-(Theophyllin-7-yl)-valeriansäureamidoxim hergestellt; F. 159 bis 162°C.

e) Ein Gemisch aus 10,0 g δ-(Theophyllin-7-yl)-valeriansäureamidoxim, 2,3 g Natriumethylat, 30 ml Ethylacetat und 30 ml Ethanol wird unter Rühren 8 h lang gekocht. Es werden 8,1 g 7-[4-(5-Methyl-1,2,4-oxadiazol-3-yl)-butan-1-yl]-theophyllin erhalten (Ausbeute 75%; F. 131 bis 132°C (Ethanol).

f) Ein Gemisch aus 4,0 g δ-(Theophyllin-7-yl)-valeriansäureamidoxim, 5,03 g β-Piperidinopropionsäureethylester, 0,51 g Natriummethylat und 70 ml Toluol wird gekocht. Mit 1,58 g Maleinsäure wird das Salz gebildet. Es werden 5,15 g 7-[4-(5-[2-Piperidinoethan-1-yl]-1,2,4-oxadiazol-3-yl)-butan-1-yl]-theophyllin-maleinat erhalten (Ausbeute 91%); F. 146 bis 147°C.

Beispiel 14

a) Durch Umsetzen von 16,8 g Theophyllinnatrium, 17,2 g 2-(5-Chlorpentan-1-yl-oxy)-pyran und 0,5 g Natriumjodid in 30 ml Dimethylformamid auf die in Beispiel 13a) beschriebene Weise werden 13,5 g 5-(Theophyllin-7-yl)-pentanol-1 erhalten (Ausbeute 61%); F. 113 bis 115°C.

b) Auf die in Beispiel 13b) beschriebene Weise werden 13,1 g 5-(Theophyllin-7-yl)-pentan-1-ol, 3,8 ml Thionylchlorid und 0,2 ml Pyridin in 60 ml Benzol umgesetzt. Es werden 11,5 g 1-Chlor-5-(theophyllin-7-yl)-pentan erhalten (Ausbeute 93%); F. 78 bis 80°C.

c) Auf die in Beispiel 13c) beschriebene Weise werden 10,0 g 1-Chlor-5-(theophyllin-7-yl)-pentan und 2,44 g Kaliumcyanid in 40 ml Dimethylformamid miteinander umgesetzt. Es werden 7,93 g 7-(5-Cyanopentan-1-yl)-theophyllin erhalten (Ausbeute 76,5%); F. 86 bis 88°C.

d) Aus dem gemäß c) hergestellten Nitril (5,5 g) werden auf die in Beispiel 12b) beschriebene Weise 5,2 g ε-(Theophyllin-7-yl)-hexansäureamidoxim hergestellt (Ausbeute 85%); F. 171 bis 174°C.

e) 3,08 g ε-(Theophyllin-7-yl)-hexansäureamidoxim werden unter schwachem Erwärmen in 10 ml Essigsäureanhydrid aufgelöst. Die Lösung wird auf dem Wasserbad 3 h lang erwärmt und dann abgekühlt. Zu der ausgeschiedenen Kristallmasse werden 10 ml Diethylether gegeben. Das Produkt wird abgesaugt und mit Ether gewaschen. Es werden 2,95 g 7-[5-(5-Methyl-1,2,4-oxadiazol-3-yl)-pentan-1-yl]-theophyllin erhalten (Ausbeute 93%); F. 160 bis 162°C.

Beispiel 15

9,42 g 3-(Theophyllin-7-yl)-2-hydroxypropionsäureamidoxim werden in eine Lösung von 1,38 g metallischem Natrium in 150 ml Ethanol eingerührt und das Gemisch nach Zugabe von 8,16 g Methylbenzoat 12 h lang auf dem Wasserbad erwärmt. Nach dem Eindampfen werden 8,5 g 7-[2-Hydroxy-3-(5-phenyl-1,2,4-oxadiazol-3-yl)-propan-1-yl]-theophyllin erhalten (Ausbeute 74%); F. 179 bis 180°C (Ethanol).

Beispiel 16

Ein Gemisch aus 5,04 g 2-(Theophyllin-7-yl)-acetamidoxim, 2,16 g Natriummethylat und 4,72 g Diethylcarbonat in 100 ml Toluol wirdc 2 h lang gekocht und dann eingedampft. Der Eindampfrückstand wird aus Wasser kristallisiert. Es werden 4,3 g 7-[(5-Hydroxy-1,2,4-oxadiazol-3-yl)-methyl]-theophyllin erhalten (Ausbeute 82%); F. 206 bis 207°C.

Beispiele 17 bis 85

Auf analoge Weise werden die in der folgenden Tabelle 2 zusammengestellten Verbindungen hergestellt

TABELLE 2

| Beispiel Nr. | A | $R_1$ | Verfahren gemäß Beispiel | F. (°C) |
|---|---|---|---|---|
| 17 | $CH_2$ | $—CH_2CH_3$ | 1f) | 129—130 |
| 18 | $CH_2$ | $—(CH_2)_2CH_3$ | 1f) | 126—127 |
| 19 | $CH_2$ | $—CH(CH_3)_2$ | 1f) | 125—126 |
| 20 | $CH_2$ | $—(CH_2)_2CH_3$ | 1f) | 128—130 |
| 21 | $CH_2$ | $—CH_2CH(CH_3)_2$ | 1f) | 124—125 |
| 22 | $CH_2$ | $—C(CH_3)_3$ | 1f) | 130—132 |
| 23 | $CH_2$ | $—(CH_2)_4CH_3$ | 1f) | 112—114 |
| 24 | $CH_2$ | $—(CH_2)_9CH_3$ | 1f) | 103—105 |
| 25 | $CH_2$ | Cyclopentyl | 1f) | 110—111 |
| 26 | $CH_2$ | Cyclohexyl | 1f) | 112—113 |
| 27 | $CH_2$ | $—CH_2NHCH(CH_3)_2$ | 3a) | 212—213 (Hydrochlorid) |
| 28 | $CH_2$ | 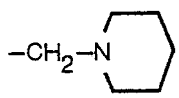 | 3a) | 203—206 (Hydrochlorid) |
| 29 | $CH_2$ | $—(CH_2)_2NHCH(CH_3)_2$ | 4a) | 212—213 (Hydrochlorid) |
| 30 | $CH_2$ | 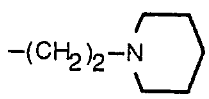 | 4b) | 96—98 |
| 31 | $CH_2$ | 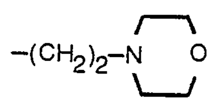 | 4b) | 108—112 |
| 32 | $CH_2$ | $—(CH_2)_2N(C_2H_5)_2$ | 4b) | 78—80; Maleinat: 127—128 |
| 33 | $CH_2$ | 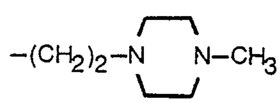 | 4b) | 87—89 |
| 34 | $CH_2$ | 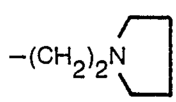 | 4b) | 203—204 Hydrochlorid |
| 35 | $CH_2$ | 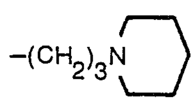 | 4b) | 206—208 Hydrochlorid |
| 36 | $CH_2$ | $—(CH_2)_3N(C_2H_5)_2$ | 4b) | 119—121 Maleinat |
| 37 | $CH_2$ | $p-Cl—C_6H_4$ | 3a) | 181—183 |
| 38 | $CH_3$ | $o-HO—C_6H_4$ | 3a) | 201—202 |
| 39 | $(CH_2)_2$ | $CH_2CH_3$ | 1f) | 113—114 |

(TABELLE 2 Fortsetzung)

| Beispiel Nr. | A | $R_1$ | Verfahren gemäß Beispiel | F. (°C) |
|---|---|---|---|---|
| 40 | $(CH_2)_2$ | $-(CH_2)_4CH_3$ | 1f) | 106—108 |
| 41 | $(CH_2)_2$ | Cyclohexyl | 1f) | 108—109 |
| 42 | $(CH_2)_2$ | $-CH_2Cl$ | 2) | 134—138 |
| 43 | $(CH_2)_2$ | $(CH_2)_2Cl$ | 4g) | 140—143 |
| 44 | $(CH_2)_2$ | $(CH_2)_3Cl$ | 4g) | 124—127 |
| 45 | $(CH_2)_2$ | $CH(OH)CH_3$ | 1f) | 129—130 |
| 46 | $(CH_2)_2$ | $CH_2N(C_2H_5)_2$ | 3a) | 203—205 Hydrochlorid |
| 47 | $(CH_2)_2$ | $(CH_2)_2NHCH(CH_3)_2$ | 4a) | 125—127 Maleinat |
| 48 | $(CH_2)_2$ | 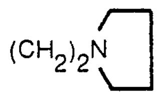 | 4a) | 207—210 Hydrochlorid |
| 49 | $(CH_2)_2$ | 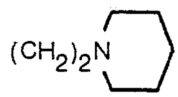 | 4a) | 204—205 Hydrochlorid |
| 50 | $(CH_2)_2$ | 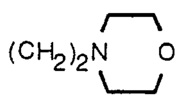 | 4a) | 180 Hydrochlorid |
| 51 | $(CH_2)_2$ | $(CH_2)_2N(C_2H_5)_2$ | 4a) | 119—120 Maleinat |
| 52 | $(CH_2)_2$ | 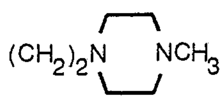 | 4a) | 165 Dimaleinat |
| 53 | $(CH_2)_2$ | 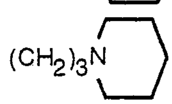 | 4a) | 203—204 Hydrochlorid |
| 54 | $(CH_2)_2$ | $(CH_2)_3N(C_2H_5)_2$ | 4a) | 200—201 Hydrochlorid |
| 55 | $(CH_2)_2$ | $C_6H_5$ | 3a) | 183—185 |
| 56 | $(CH_2)_2$ | p-Cl—$C_6H_4$ | 3a) | 190—191 |
| 57 | $(CH_2)_2$ | o-HO—$C_6H_4$ | 3a) | 202—205 |
| 58 | $(CH_2)_2$ | —OH | 1b) | 205—206 |
| 59 | $(CH_2)_3$ | $CH_3$ | 1d) | 120—122 |
| 60 | $(CH_2)_3$ | Cyclohexyl | 1f) | 97—98 |
| 61 | $(CH_2)_3$ | $(CH_2)_2Cl$ | 4g) | 111—112 |
| 62 | $(CH_2)_3$ | 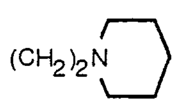 | 12c) | 158—159 Maleinat |

(TABELLE 2 Fortsetzung)

| Beispiel Nr. | A | $R_1$ | Verfahren gemäß Beispiel | F. (°C) |
|---|---|---|---|---|
| 63 | $(CH_2)_3$ | 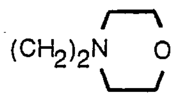 | 12c) | 134—136 Maleinat |
| 64 | $(CH_2)_3$ | $(CH_2)_2NHCH(CH_3)_2$ | 12c) | 128—130 Maleinat |
| 65 | $(CH_2)_3$ | 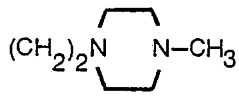 | 12c) | 171—172 Dimaleinat |
| 66 | $(CH_2)_3$ | 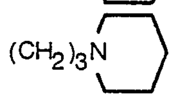 | 12c) | 203—205 Hydrochlorid |
| 67 | $(CH_2)_3$ | —OH | 16) | 212—214 |
| 68 | $(CH_2)_3$ | $—C_6H_5$ | 3a) | 176—178 |
| 69 | $(CH_2)_4$ | $(CH_2)_2N(C_2H_5)_2$ | 13f) | 144—146 Maleinat |
| 70 | $(CH_2)_5$ | $(CH_2)_2N(C_2H_5)_2$ | 13f) | 148—150 |
| 71 | $CH_2CH(OH)CH_2$ | $CH_2CH_3$ | 1f) | 146—148 |
| 72 | $CH_2CH(OH)CH_2$ | $(CH_2)_2CH_3$ | 1f) | 140—141 |
| 73 | $CH_2CH(OH)CH_2$ | Cyclohexyl | 1f) | 133—135 |
| 74 | $CH_2CH(OH)CH_2$ | $(CH_2)_2N(C_2H_5)$ | 11) | 143 Maleinat |
| 75 | $CH_2CH(OH)CH_2$ | 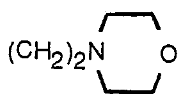 | 11) | 156—159 Maleinat |
| 76 | $CH_2CH(OH)CH_2$ | 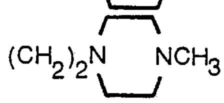 | 11) | 149—150 Dimaleinat |
| 77 | $CH_2CH(OH)CH_2$ | $(CH_2)_2NHCH(CH_3)_2$ | 11) | 207 Hydrochlorid |
| 78 | $CH_2CH(OH)CH_2$ | $—o\text{-}HO—C_6H_4$ | 3a) | 206—207 |
| 79 | $CH_2CH(OH)CH_2$ | —OH | 16) | 200—203 |
| 80 | $CH_2$ | $CH_2—C_6H_5$ | 3a) | 142—145 |
| 81 | $(CH_2)_2$ | $CH_2—C_6H_5$ | 3a) | 136—138 |
| 82 | $(CH_2)_2$ | $CH_2CH(C_6H_5)_2$ | 3a) | 181—182 |
| 83 | $(CH_2)_3$ | $CH_2CH(C_6H_5)_2$ | 3a) | 134—135 |
| 84 | $CH_2CH(OH)CH_2$ | $—CH_2—C_6H_5$ | 3a) | 146—148 |
| 85 | $(CH_2)_3$ | $—CH_2—C_6H_5$ | 3a) | 96—98 |

Beispiel 86

5,04 g 2-(Theophyllin-7-yl)-acetamidoxim und 1,08 g Natriummethylat werden in 60 ml Dimethylformamid suspendiert; die Suspension wird unter Rühren innerhalb von 15 min mit einer Lösung von 5,13 g Theophyllin-7-yl-acetylchlorid in 25 ml Dimethylformamid versetzt. Das Gemisch wird 1 h lang gekocht, mit wenig Aktivkohle geklärt und heiß filtriert; das Filtrat wird unter vermindertem Druck eingedampft. Der Rückstand wird in 100 ml 0,1 n Natriumhydrogencarbonatlösung heiß gelöst. Nach dem Abkühlen werden die Kristalle abgetrennt, mit Wasser gewaschen und aus 50-%igem wäßrigen Ethanol umkristallisiert. Es werden 6,6 g 3-(Theophyllin-7-yl-methyl)-5-(theophyllin-7-ylmethyl)-1,2,4-oxadiazol erhalten; F. 271 bis 272°C.

Arzneimittelpräparate

Beispiel 87

a) Tabletten

| | (g) |
|---|---|
| 7-[(5-Methyl-1,2,4-oxadiazol-3-yl)-methyl]-theophyllin | 100,0 |
| Maisstärke | 130,0 |
| Calciumphosphat | 209,0 |
| Magnesiumstearat | 1,0 |
| | 440,0 |

Die miteinander vermischten Bestandteile werden in bekannter Weise granuliert und zu 1000 Stück Tabletten einer Masse von je 440 mg verpreßt. Jede Tablette enthält 100 mg Wirkstoff.

b) Injektionslösung

200,0 g 7-[(5-[2-Diethylaminoethan-1-yl]-1,2,4-oxadiazol-3-yl)-methyl]-theophyllin-maleinat werden mit destilliertem Wasser auf 1 l aufgefüllt. Die wäßrige Wirkstofflösung wird in bekannter Weise behandelt und in 1000 Ampullen abgefüllt. Jede Ampulle enthält in 1 ml Injektionslösung 200 mg Wirkstoff.

c) Dragees

| | (g) |
|---|---|
| 7-[(5-Methyl-1,2,4-oxadiazol-3-yl)-methyl]-theophyllin | 150,0 |
| Carboxymethylcellulose | 300,0 |
| Stearinsäure | 20,0 |
| Celluloseacetatphthalat | 30,0 |
| | 500,0 |

Der Wirkstoff wird mit der Carboxymethylcellulose und der Stearinsäure vermischt; das Gemisch wird mit der Lösung des Celluloseacetatphthalats in 200 ml Ethylacetat/Ethanol granuliert. Aus dem Granulat werden Kerne von je 500 mg Masse gepreßt, die in an sich bekannter Weise mit zuckerhaltiger 5-%iger wäßriger Polyvinylpyrrolidonlösung überzogen werden. Jedes Dragee enthält 150 mg Wirkstoff.

**Patentansprüche**

1. 7-Substituierte Theophyllinderivate der allgemeinen Formel I

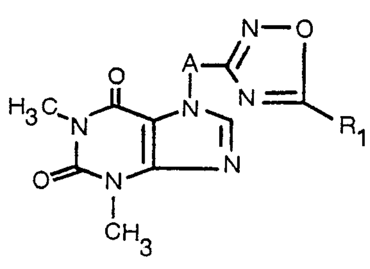

(I),

worin bedeuten:

A geradkettiges oder verzweigtes $C_{1-5}$-Alkylen oder

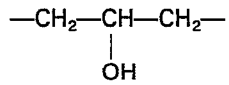

und

$R_1$ Hydroxy, geradkettiges oder verzweigtes $C_{1-10}$-Alkyl, -Halogenalkyl oder -Hydroxyalkyl, $C_{5-6}$-Cycloalkyl, Vinyl, 2-Ethoxyethyl, $C_{2-5}$-Carbonylalkyl, Benzyl, 2,2-Diphenylethyl, Theophyllin-7-ylmethyl, eine Aminoalkylgruppe der allgemeinen Formel 1,

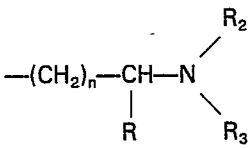

(1),

oder eine Phenylgruppe der allgemeinen Formel 2,

$$R_4R_5C_6H_3-\qquad (2),$$

wobei in Formel 1 bzw. 2 bedeuten:

R H oder Methyl,

$R_2$ und $R_3$ H oder geradkettiges oder verzweigtes $C_{1-4}$-Alkyl oder $R_2$ und $R_3$ zusammen mit dem N-Atom einen 5- oder 6-gliedrigen Heterocyclus, der ein gegebenenfalls durch Methyl substituiertes weiteres Stickstoffatom oder ein Sauerstoffatom enthalten kann,

n 0, 1, 2 oder 3 und

$R_4$ und $R_5$ unabhängig H, Chlor, Hydroxy, Methoxy, Ethoxy, Methyl oder Amino, sowie ihre physiologisch verträglichen Säureadditionssalze.

2. Verbindungen der allgemeinen Formel I nach Anspruch 1, worin bedeuten:

A $C_{1-3}$-Alkylen oder

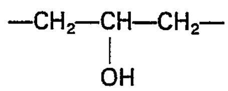

und

$R_1$ $C_{1-4}$-Alkyl oder eine Aminoalkylgruppe der allgemeinen Formel 1 sowie ihre Säureadditionssalze.

3. 7-[(5-Methyl-1,2,4-oxadiazol-3-yl)-methyl]-theophyllin;
7-[(5-Ethyl-1,2,4-oxadiazol-3-yl)-methyl]-theophyllin;
7-[(5-[Propan-2-yl]-1,2,4-oxadiazol-3-yl)-methyl]-theophyllin;
7-[(5-[Butan-1-yl]-1,2,4-oxadiazol-3-yl)-methyl]-theophyllin;
7-[(5-[2-Methyl-propan-1-yl]-1,2,4-oxadiazol-3-yl)-methyl]-theophyllin;
7-[(5-[1,1-Dimethylethan-1-yl]-1,2,4-oxadiazol-3-yl)-methyl]-theophyllin;
7-[(5-Pentyl-1,2,4-oxadiazol-3-yl)-methyl]-theophyllin;
7-[(5-Decyl-1,2,4-oxadiazol-3-yl)-methyl]-theophyllin;
7-[(5-Cyclopentyl-1,2,4-oxadiazol-3-yl)-methyl]-theophyllin;
7-[(5-Cyclohexanyl-1,2,4-oxadiazol-3-yl)-methyl]-theophyllin;
7-[(5-[1-Hydroxyethyl]-1,2,4-oxadiazol-3-yl)-methyl]-theophyllin;
7-[(5-Chlormethyl-1,2,4-oxadiazol-3-yl)-methyl]-theophyllin;
7-[(5-[3-Chlorpropyl]-1,2,4-oxadiazol-3-yl)-methyl]-theophyllin;
7-[(5-[3-Oxobutyl]-1,2,4-oxadiazol-3-yl)-methyl]-theophyllin;
7-[(5-[2-Chlorethyl]-1,2,4-oxadiazol-3-yl)-methyl]-theophyllin;
7-[(5-[2-Ethoxyethyl]-1,2,4-oxadiazol-3-yl)-methyl]-theophyllin;
7-[(5-Isopropylaminomethyl-1,2,4-oxadiazol-3-yl)-methyl]-theophyllin;
7-[(5-[2-N-Methylpiperazinoethyl]-1,2,4-oxadiazol-3-yl)-methyl]-theophyllin;
7-[(5-[2-Pyrrolidinoethyl]-1,2,4-oxadiazol-3-yl)-methyl]-theophyllin;
7-[(5-[3-Piperidinopropyl]-1,2,4-oxadiazol-3-yl)-methyl]-theophyllin;
7-[(5-[3-Diethylaminopropyl]-1,2,4-oxadiazol-3-yl)-methyl]-theophyllin;
7-[(5-[3-Isopropylaminobutyl]-1,2,4-oxadiazol-3-yl)-methyl]-theophyllin;
7-[(5-[4-Diethylaminobutyl]-1,2,4-oxadiazol-3-yl)-methyl]-theophyllin;
7-[(5-Phenyl-1,2,4-oxadiazol-3-yl)-methyl]-theophyllin;
7-[(5-[4-Chlorphenyl]-1,2,4-oxadiazol-3-yl)-methyl]-theophyllin;

7-[(5-[2-Hydroxyphenyl]-1,2,4-oxadiazol-3-yl)-methyl]-theophyllin;
7-[(5-Hydroxy-1,2,4-oxadiazol-3-yl)-methyl]-theophyllin;
7-[(5-Diethylaminomethyl-1,2,4-oxadiazol-3-yl)-methyl]-theophyllin;
7-[(5-Piperidinomethyl-1,2,4-oxadiazol-3-yl)-methyl]-theophyllin;
7-[(5-Aminomethyl-1,2,4-oxadiazol-3-yl)-methyl]-theophyllin;
7-[(5-Dimethylaminomethyl-1,2,4-oxadiazol-3-yl)-methyl]-theophyllin;
7-[(5-[2-Isopropylaminoethyl]-1,2,4-oxadiazol-3-yl)-methyl]-theophyllin;
7-[(5-[2-Piperidinoethyl]-1,2,4-oxadiazol-3-yl)-methyl]-theophyllin;
7-[(5-[2-Morpholinoethyl]-1,2,4-oxadiazol-3-yl)-methyl]-theophyllin;
7-[(5-[2-Diethylaminoethyl]-1,2,4-oxadiazol-3-yl)-methyl]-theophyllin;
7-[(5-Benzyl-1,2,4-oxadiazol-3-yl)-methyl]-theophyllin; und
7-[(5-[Theophyllin-7-ylmethyl]-1,2,4-oxadiazol-3-yl)-methyl]-theophyllin;
4. 7-[2-(5-Methyl-1,2,4-oxadiazol-3-yl)-ethyl]-theophyllin;
7-[2-(5-Ethyl-1,2,4-oxadiazol-3-yl)-ethyl]-theophyllin;
7-[2-(5-Pentyl-1,2,4-oxadiazol-3-yl)-ethyl]-theophyllin;
7-[2-(5-Cyclohexyl-1,2,4-oxadiazol-3-yl)-ethyl]-theophyllin;
7-[2-(5-(2-Chlorethyl)-1,2,4-oxadiazol-3-yl)-ethyl]-theophyllin;
7-[2-(5-Chlormethyl-1,2,4-oxadiazol-3-yl)-ethyl]-theophyllin;
7-[2-(5-(3-Chlorpropyl]-1,2,4-oxadiazol-3-yl)-ethyl]-theophyllin;
7-[2-(5-[1-Hydroxyethyl]-1,2,4-oxadiazol-3-yl)-ethyl]-theophyllin;
7-[2-(5-Diethylaminomethyl-1,2,4-oxadiazol-3-yl)-ethyl]-theophyllin;
7-[2-(5-[2-Isopropylaminoethyl]-1,2,4-oxadiazol-3-yl)-ethyl]-theophyllin;
7-[2-(5-[2-Pyrrolidinoethyl]-1,2,4-oxadiazol-3-yl)-ethyl]-theophyllin;
7-[2-(5-[2-Piperidinoethyl]-1,2,4-oxadiazol-3-yl)-ethyl]-theophyllin;
7-[2-(5-[2-Morpholinoethyl]-1,2,4-oxadiazol-3-yl)-ethyl]-theophyllin;
7-[2-(5-[2-Diethylaminoethyl]-1,2,4-oxadiazol-3-yl)-ethyl]-theophyllin;
7-[2-(5-[2-N-Methylpiperazinoethyl]-1,2,4-oxadiazol-3-yl)-ethyl]-theophyllin;
7-[2-(5-[3-Piperidinopropyl]-1,2,4-oxadiazol-3-yl)-ethyl]-theophyllin;
7-[2-(5-[3-Diethylaminopropyl]-1,2,4-oxadiazol-3-yl)-ethyl]-theophyllin;
7-[2-(5-Phenyl-1,2,4-oxadiazol-3-yl)-ethyl]-theophyllin;
7-[2-(5-Hydroxy-1,2,4-oxadiazol-3-yl)-ethyl]-theophyllin;
7-[2-(5-Chlorphenyl]-1,2,4-oxadiazol-3-yl)-ethyl]-theophyllin;
7-[2-(5-[2-Hydroxyphenyl]-1,2,4-oxadiazol-3-yl)-ethyl]-theophyllin;
7-[2-(5-Benzyl-1,2,4-oxadiazol-3-yl)-ethyl]-theophyllin; und
7-[2-(5-[2,2-Diphenylethyl]-1,2,4-oxadiazol-3-yl)-ethyl]-theophyllin.
5. 7-[3-(5-Methyl-1,2,4-oxadiazol-3-yl)-propyl]-theophyllin;
7-[3-(5-Cyclohexyl-1,2,4-oxadiazol-3-yl)-propyl]-theophyllin;
7-[3-(5-[2-Chlorethyl]-1,2,4-oxadiazol-3-yl)-propyl]-theophyllin;
7-[3-(5-[2-Piperidinoethyl]-1,2,4-oxadiazol-3-yl)-propyl]-theophyllin;
7-[3-(5-[2-Diethylaminoethyl]-1,2,4-oxadiazol-3-yl)-propyl]-theophyllin;
7-[3-(5-[2-Morpholinoethyl]-1,2,4-oxadiazol-3-yl)-propyl]-theophyllin;
7-[3-(5-[2-Isopropylaminoethyl]-1,2,4-oxadiazol-3-yl)-propyl]-theophyllin;
7-[3-(5-[2-N-Methylpiperazinoethyl]-1,2,4-oxadiazol-3-yl)-propyl]-theophyllin;
7-[3-(5-[3-Piperidinopropyl]-1,2,4-oxadiazol-3-yl)-propyl]-theophyllin;
7-[3-(5-Hydroxy-1,2,4-oxadiazol-3-yl)-propyl]-theophyllin;
7-[3-(5-Phenyl-1,2,4-oxadiazol-3-yl)-propyl]-theophyllin;
7-[2-Hydroxy-3-(5-methyl-1,2,4-oxadiazol-3-yl)-propyl]-theophyllin;
7-[2-Hydroxy-3-(5-ethyl-1,2,4-oxadiazol-3-yl)-propyl]-theophyllin;
7-[2-Hydroxy-3-(5-cyclohexyl-1,2,4-oxadiazol-3-yl)-propyl]-theophyllin;
7-[2-Hydroxy-3-(5-n-propyl-1,2,4-oxadiazol-3-yl)-propyl]-theophyllin;
7-[2-Hydroxy-3-(5-[2-diethylaminoethyl]-1,2,4-oxadiazol-3-yl)-propyl]-theophyllin;
7-[2-Hydroxy-3-(5-[2-Piperidinoethyl]-1,2,4-oxadiazol-3-yl)-propyl]-theophyllin;
7-[2-Hydroxy-3-(5-[2-morpholinoethyl]-1,2,4-oxadiazol-3-yl)-propyl]-theophyllin;
7-[2-Hydroxy-3-(5-[2-N-methylpiperazinoethyl]-1,2,4-oxadiazol-3-yl)-propyl]-theophyllin;
7-[2-Hydroxy-3-(5-[2-isopropylaminoethyl]-1,2,4-oxadiazol-3-yl)-propyl]-theophyllin;
7-[2-Hydroxy-3-(5-phenyl-1,2,4-oxadiazol-3-yl)-propyl]-theophyllin;
7-[2-Hydroxy-3-(5-[2-hydroxyphenyl]-1,2,4-oxadiazol-3-yl)-propyl]-theophyllin;
7-[2-Hydroxy-3-(5-hydroxy-1,2,4-oxadiazol-3-yl)-propyl]-theophyllin;
7-[3-(5-Benzyl-1,2,4-oxadiazol-3-yl)-propyl]-theophyllin; und
7-[3-(5-[2,2-Diphenylethyl]-1,2,4-oxadiazol-3-yl)-propyl]-theophyllin.
6. 7-[4-(5-Methyl-1,2,4-oxadiazol-3-yl)-butyl]-theophyllin;
7-[4-(5-[2-Piperidinoethyl]-1,2,4-oxadiazol-3-yl)-butyl]-theophyllin;
7-[5-[2-Diethylaminoethyl]-1,2,4-oxadiazol-3-yl)-pentyl]-theophyllin;
7-[4-(5-(5-Methyl-1,2,4-oxadiazol-3-yl)-pentyl]-theophyllin; und
7-[5-(5-[2-Diethylaminoethyl]-1,2,4-oxadiazol-3-yl)-pentyl]-theophyllin.

7. Verfahren zur Herstellung der Theophyllinderivate der Formel I nach einem der Ansprüche 1 bis 6, gekennzeichnet durch

(A) Umsetzung von Amidoximen der allgemeinen Formel II

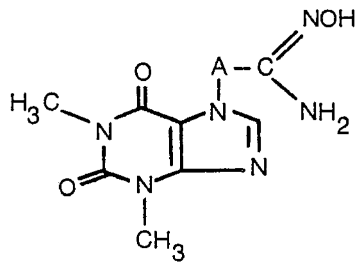

(II)

mit A wie in Anspruch 1 mit Säuren der allgemeinen Formel III

$$R_6\text{—COOH} \qquad (III),$$

worin $R_6$ dasselbe wie $R_1$ in Anspruch 1 oder eine in $R_1$ überführbare Gruppe bedeutet, und/oder mit acylierend wirkenden Derivaten der Säuren der allgemeinen Formel III unmittelbar zu Verbindungen der allgemeinen Formel Ic

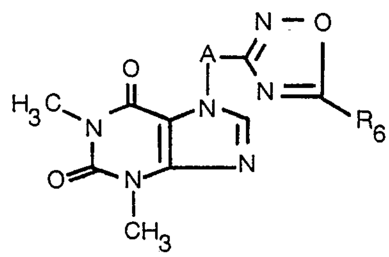

(Ic)

mit A und $R_6$ wie oben und erforderlichenfalls Umwandlung von $R_6$ in $R_1$ oder

(B) Umsetzung von Amidoximen der allgemeinen Formel II mit Säuren der allgemeinen Formel III oder acylierend wirkenden Derivaten der Säuren der allgemeinen Formel III zu acylierten Derivaten der allgemeinen Formel IV

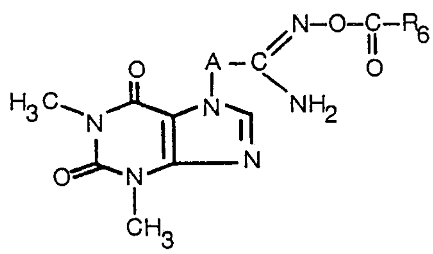

(IV)

mit A und $R_6$ wie oben und Cyclisierung der Verbindungen der allgemeinen Formel IV, gegebenenfalls nach Isolierung, unter Wasserabspaltung und erforderlichenfalls Umwandlung von $R_6$ in $R_1$ oder

(C) Umsetzung von Verbindungen der allgemeinen Formel V

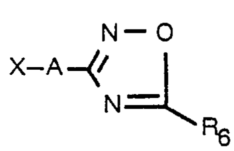

(V)

mit A und $R_6$ wie oben und X = Halogen oder eine Sulfonsäureestergruppe mit Theophyllin in Gegenwart eines basischen Katalysators oder mit einem mit einer Base gebildeten Salz des Theophyllins und erforderlichenfalls Umwandlung von $R_6$ in $R_1$ oder

(D) Umsetzung von Olefinen der allgemeinen Formel VI

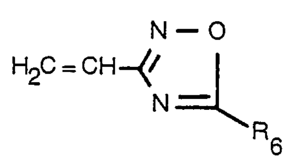

(VI)

mit $R_6$ wie oben in Gegenwart eines basischen Katalysators mit Theophyllin zu Verbindungen der allgemeinen Formel Id

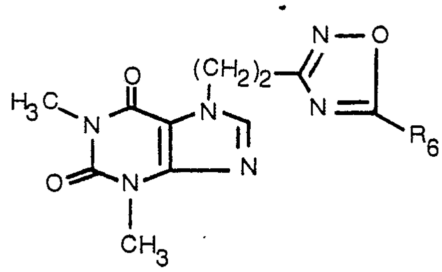

(Id)

mit $R_6$ wie oben und erforderlichenfalls Umwandlung von $R_6$ in $R_1$ oder

(E) Umsetzung von Epoxiden der allgemeinen Formel VII

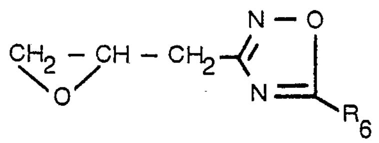

(VII)

mit $R_6$ wie oben mit Theophyllin zu einer Verbindung der Formel I mit

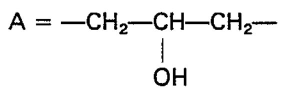

und erforderlichenfalls Umwandlung von $R_6$ in $R_1$.

8. Verfahren nach Anspruch 7 zur Herstellung von Verbindungen der allgemeinen Formel I, in der $R_1$ eine Aminoalkylgruppe der allgemeinen Formel 1 wie in Anspruch 1 oder

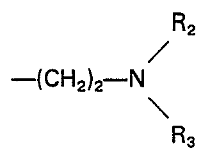

mit $R_2$ und $R_3$ wie in Anspruch 1 bedeutet, gekennzeichnet durch

(a) Umsetzung von Verbindungen der allgemeinen Formeln I oder IV, worin $R_6$ Vinyl, 2-Ethoxyethyl, $C_{1-4}$-Halogenalkyl, O-Mesylalkyl oder O-Tosylalkyl und A dasselbe wie in Anspruch 1 bedeuten, mit einem Amin der allgemeinen Formel $HNR_2R_3$ mit $R_2$ und $R_3$ wie in Anspruch 1 oder

(b) reduktive Kondensation von Verbindungen der allgemeinen Formel I oder IV, worin $R_6$ $C_{1-4}$-Oxoalkyl und A dasselbe wie in Anspruch 1 bedeuten, mit einem Amin der allgemeinen Formel $R_8NH_2$, worin $R_8$ dasselbe wie $R_2$ mit Ausnahme von Wasserstoff bedeutet, oder

(c) Alkylierung von Verbindungen der allgemeinen Formel I oder IV, worin $R_6$ $-(CH_2)_n-NH_2$ mit $n = 1$, 2, 3 oder 4 und A dasselbe wie in Anspruch 1 bedeuten.

9. Verfahren nach Anspruch 7, oder 8, gekennzeichnet durch Verwendung von Verbindungen der Formeln III, IV, V, VI oder VII, in denen $R_6$ Vinyl, 2-Ethoxyethyl, $C_{3-4}$-Oxoalkyl, O-Tosylalkyl, O-Mesylalkyl oder Halogenalkyl bedeutet.

10. Verfahren nach Anspruch 9, gekennzeichnet durch Verwendung von Verbindungen der Formel IV, in der $R_6$ Chloralkyl oder Bromalkyl bedeutet.

11. Arzneimittel, gekennzeichnet durch Verbindungen der allgemeinen Formel I nach einem der Ansprüche 1 bis 6 als Wirkstoffe neben üblichen Hilfs- und/oder Trägerstoffen.

12. Arzneimittel nach Anspruch 11, ferner gekennzeichnet durch Gehalt an weiteren biologisch aktiven Stoffen.

**Revendications**

1. Dérivés de la théophylline, substitués en position 7, répondant à la formule générale I

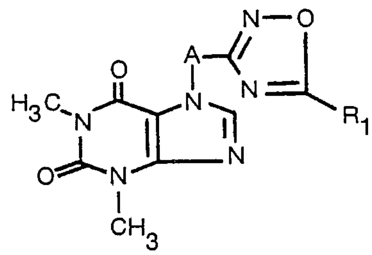

(I),

dans laquelle

A représente un groupe alkylène à chaîne droite ou ramifiée en C1—C5 ou un groupe

$$-CH_2-\underset{\displaystyle OH}{\underset{|}{CH}}-CH_2-$$

et

$R_1$ représente un groupe hydroxy, alkyle, halogénoalkyle ou hydroxyalkyle à chaîne droite ou ramifiée en C1—C10, cycloalkyle en C5—C6, vinyle, 2-éthoxyéthyle, carbonylalkyle en C2—C5, benzyle, 2,2-diphényléthyle, théophylline-7-ylméthyle, un groupe aminoalkyle de formule générale 1,

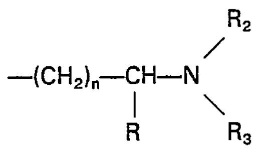

(1),

ou un groupe phényle de formule générale 2

$$R_4R_5C_6H_3- \qquad (2),$$

les symboles des formules 1 et 2 ayant les significations suivantes:

R représente H ou un groupe méthyle,

$R_2$ et $R_3$ représentent H ou des groupes alkyle à chaîne droite ou ramifiée en C1—C4 ou bien $R_2$ et $R_3$ forment ensemble et avec l'atome d'azote un hétérocycle à 5 ou 6 chaînons qui peut contenir un autre atome d'azote éventuellement substitué par un groupe méthyle ou un atome d'oxygène,

n est égal à 0, 1, 2 ou 3 et

$R_4$ et $R_5$ représentent chacun, indépendamment l'un de l'autre, H, le chlore, un groupe hydroxy, méthoxy, éthoxy, méthyle ou amino,

et leurs sels acceptables pour l'usage pharmaceutique formés par addition avec des acides.

2. Composés de formule générale I selon la revendication 1, dans laquelle

A représente un groupe alkylène en C1—C3 ou

$$-CH_2-\underset{\displaystyle OH}{\underset{|}{CH}}-CH_2-$$

et

$R_1$ représente un groupe alkyle en C1—C4 ou un groupe aminoalkyle de formule générale 1 et leurs sels formés par addition avec des acides.

3. La 7-[(5-méthyl-1,2,4-oxadiazole-3-yl)-méthyl]-théophylline;
la 7-[(5-éthyl-1,2,4-oxadiazole-3-yl)-méthyl]-théophylline;
la 7-[(5-[propane-2-yl]-1,2,4-oxadiazole-3-yl)-méthyl]-théophylline;
la 7-[(5-[butane-1-yl]-1,2,4-oxadiazole-3-yl)-méthyl]-théophylline;
la 7-[(5-[2-méthyl-propane-1-yl]-1,2,4-oxadiazole-3-yl)-méthyl]-théophylline;
la 7-[(5-[1,1-diméthyléthane-1-yl]-1,2,4-oxadiazole-3-yl)-méthyl]-théophylline;
la 7-[(5-pentyl-1,2,4-oxadiazole-3-yl)-méthyl]-théophylline;
la 7-[(5-décyl-1,2,4-oxadiazole-3-yl)-méthyl]-théophylline;
la 7-[(5-cyclopentyl-1,2,4-oxadiazole-3-yl)-méthyl]-théophylline;
la 7-[(5-cyclohexanyl-1,2,4-oxadiazole-3-yl)-méthyl]-théophylline;

la 7-[(5-[1-hydroxyéthyl]-1,2,4-oxadiazole-3-yl)-méthyl]-théophylline;
la 7-[(5-chlorométhyl-1,2,4-oxadiazole-3-yl)-méthyl]-théophylline;
la 7-[(5-[3-chloropropyl]-1,2,4-oxadiazole-3-yl)-méthyl]-théophylline;
la 7-[(5-oxobutyl]-1,2,4-oxadiazole-3-yl)-méthyl]-théophylline;
la 7-[(5-[2-chloréthyl]-1,2,4-oxadiazole-3-yl)-méthyl]-théophylline;
la 7-[(5-[2-éthoxyéthyl]-1,2,4-oxadiazole-3-yl)-méthyl]-théophylline;
la 7-[(5-isopropylaminométhyl-1,2,4-oxadiazole-3-yl)-méthyl]-théophylline;
la 7-[(5-[2-N-méthylpipérazinoéthyl]-1,2,4-oxadiazole-3-yl)-méthyl]-théophylline;
la 7-[(5-[2-pyrrolidinoéthyl]-1,2,4-oxadiazole-3-yl)-méthyl]-théophylline;
la 7-[(5-[3-pipéridinopropyl]-1,2,4-oxadiazole-3-yl)-méthyl]-théophylline;
la 7-[(5-[3-diéthylaminopropyl]-1,2,4-oxadiazole-3-yl)-méthyl]-théophylline;
la 7-[(5-[3-isopropylaminobutyl]-1,2,4-oxadiazole-3-yl)-méthyl]-théophylline;
la 7-[(5-[4-diéthylaminobutyl]-1,2,4-oxadiazole-3-yl)-méthyl]-théophylline;
la 7-[(5-phényl-1,2,4-oxadiazole-3-yl)-méthyl]-théophylline;
la 7-[(5-[4-chlorophényl]-1,2,4-oxadiazole-3-yl)-méthyl]-théophylline;
la 7-[(5-[2-hydroxyphényl]-1,2,4-oxadiazole-3-yl)-méthyl]-théophylline;
la 7-[(5-hydroxy-1,2,4-oxadiazole-3-yl)-méthyl]-théophylline;
la 7-[(5-diéthylaminométhyl-1,2,4-oxadiazole-3-yl)-méthyl]-théophylline;
la 7-[(5-pipéridinométhyl-1,2,4-oxadiazole-3-yl)-méthyl]-théophylline;
la 7-[(5-aminométhyl-1,2,4-oxadiazole-3-yl)-méthyl]-théophylline;
la 7-[(5-diméthylaminométhyl-1,2,4-oxadiazole-3-yl)-méthyl]-théophylline;
la 7-[(5-[2-isopropylaminoéthyl]-1,2,4-oxadiazole-3-yl)-méthyl]-théophylline;
la 7-[(5-[2-pipéridinoéthyl]-1,2,4-oxadiazole-3-yl)-méthyl]-théophylline;
la 7-[(5-[2-morpholinoéthyl]-1,2,4-oxadiazole-3-yl)-méthyl]-théophylline;
la 7-[(5-[2-diéthylaminoéthyl]-1,2,4-oxadiazole-3-yl)-méthyl]-théophylline;
la 7-[(5-benzyl-1,2,4-oxadiazole-3-yl)-méthyl]-théophylline et
la 7-[(5-[théophylline-7-ylméthyl]-1,2,4-oxadiazole-3-yl)-méthyl]-théophylline.

4. La 7-[2-(5-méthyl-1,2,4-oxadiazole-3-yl)-éthyl]-théophylline;
la 7-[2-(5-éthyl-1,2,4-oxadiazole-3-yl)-éthyl]-théophylline;
la 7-[2-(5-pentyl-1,2,4-oxadiazole-3-yl)-éthyl]-théophylline;
la 7-[2-(5-(2-cyclohexyl-1,2,4-oxadiazole-3-yl)-éthyl]-théophylline;
la 7-[2-(5-(2-chloroéthyl)-1,2,4-oxadiazole-3-yl)-éthyl]-théophylline;
la 7-[2-(5-chlorométhyl-1,2,4-oxadiazole-3-yl)-éthyl]-théophylline;
la 7-[2-(5-[3-chloropropyl]-1,2,4-oxadiazole-3-yl)-éthyl]-théophylline;
la 7-[2-(5-[1-hydroxyéthyl]-1,2,4-oxadiazole-3-yl)-éthyl]-théophylline;
la 7-[2-(5-diéthylaminométhyl-1,2,4-oxadiazole-3-yl)-éthyl]-théophylline;
la 7-[2-(5-[2-isopropylaminoéthyl]-1,2,4-oxadiazole-3-yl)-éthyl]-théophylline;
la 7-[2-(5-[2-pyrrolidinoéthyl]-1,2,4-oxadiazole-3-yl)-éthyl]-théophylline;
la 7-[2-(5-[2-pipéridinoéthyl]-1,2,4-oxadiazole-3-yl)-éthyl]-théophylline;
la 7-[2-(5-[2-morpholinoéthyl]-1,2,4-oxadiazole-3-yl)-éthyl]-théophylline;
la 7-[2-(5-[2-diéthylaminoéthyl]-1,2,4-oxadiazole-3-yl)-éthyl]-théophylline;
la 7-[2-(5-[2-N-méthylpipérazinoéthyl]-1,2,4-oxadiazole-3-yl)-éthyl]-théophylline;
la 7-[2-(5-[3-pipéridinopropyl]-1,2,4-oxadiazole-3-yl)-éthyl]-théophylline;
la 7-[2-(5-[3-diéthylaminopropyl]-1,2,4-oxadiazole-3-yl)-éthyl]-théophylline;
la 7-[2-(5-phényl-1,2,4-oxadiazole-3-yl)-éthyl]-théophylline;
la 7-[2-(5-hydroxy-1,2,4-oxadiazole-3-yl)-éthyl]-théophylline;
la 7-[2-(5-[4-chlorophényl]-1,2,4-oxadiazole-3-yl)-éthyl]-théophylline;
la 7-[2-(5-[2-hydroxyphényl]-1,2,4-oxadiazole-3-yl)-éthyl]-théophylline;
la 7-[2-(5-benzyl-1,2,4-oxadiazole-3-yl)-éthyl]-théophylline et
la 7-[2-(5-[2,2-diphényléthyl]-1,2,4-oxadiazole-3-yl)-éthyl]-théophylline.

5. La 7-[3-(5-méthyl-1,2,4-oxadiazole-3-yl)-propyl]-théophylline;
la 7-[3-(5-cyclohexyl-1,2,4-oxadiazole-3-yl)-propyl]-théophylline;
la 7-[3-(5-[2-chloroéthyl]-1,2,4-oxadiazole-3-yl)-propyl]-théophylline;
la 7-[3-(5-[2-pipéridinoéthyl]-1,2,4-oxadiazole-3-yl)-propyl]-théophylline;
la 7-[3-(5-[2-diéthylaminoéthyl]-1,2,4-oxadiazole-3-yl)-propyl]-théophylline;
la 7-[3-(5-[2-morphlinoéthyl]-1,2,4-oxadiazole-3-yl)-propyl]-théophylline;
la 7-[3-(5-[2-isopropylaminoéthyl]-1,2,4-oxadiazole-3-yl)-propyl]-théophylline;
la 7-[3-(5-[2-N-méthylpipérazinoéthyl]-1,2,4-oxadiazole-3-yl)-propyl]-théophylline;
la 7-[3-(5-[3-pipéridinopropyl]-1,2,4-oxadiazole-3-yl)-propyl]-théophylline;
la 7-[3-(5-hydroxy-1,2,4-oxadiazole-3-yl)-propyl]-théophylline;
la 7-[3-(5-phényl-1,2,4-oxadiazole-3-yl)-propyl]-théophylline;
la 7-[2-hydroxy-3-(5-méthyl-1,2,4-oxadiazole-3-yl)-propyl]-théophylline;
la 7-[2-hydroxy-3-(5-éthyl-1,2,4-oxadiazole-3-yl)-propyl]-théophylline;
la 7-[2-hydroxy-3-(5-cyclohexyl-1,2,4-oxadiazole-3-yl)-propyl]-théophylline;
la 7-[2-hydroxy-3-(5-n-propyl-1,2,4-oxadiazole-3-yl)-propyl]-théophylline;

la 7-[2-hydroxy-3-(5-[2-diéthylaminoéthyl]-1,2,4-oxadiazole-3-yl)-propyl]théophylline;
la 7-[2-hydroxy-3-(5-[2-pipéridinoéthyl]-1,2,4-oxadiazole-3-yl)-propyl]-théophylline;
la 7-[2-hydroxy-3-(5-[2-morpholinoéthyl]-1,2,4-oxadiazole-3-yl)-propyl]-théophylline;
la 7-[2-hydroxy-3-(5-[2-N-méthylpipérazinoéthyl]-1,2,4-oxadiazole-3-yl)-propyl]-théophylline;
la 7-[2-hydroxy-3-(5-[2-isopropylaminoéthyl]-1,2,4-oxadiazole-3-yl)-propyl]-théophylline;
la 7-[2-hydroxy-3-(5-phényl-1,2,4-oxadiazole-3-yl)-propyl]-théophylline;
la 7-[2-hydroxy-3-(5-[2-hydroxyphényl]-1,2,4-oxadiazole-3-yl)-propyl]-théophylline;
la 7-[2-hydroxy-3-(5-hydroxy-1,2,4-oxadiazole-3-yl)-propyl]-théophylline;
la 7-[3-(5-benzyl-1,2,4-oxadiazole-3-yl)-propyl]-théophylline et
la 7-[3-(5-[2,2-diphényléthyl]-1,2,4-oxadiazole-3-yl)-propyl]-théophylline.

6. La 7-[4-(5-méthyl-1,2,4-oxadiazole-3-yl)-butyl]-théophylline;
la 7-[4-(5-(2-pipéridinoéthyl]-1,2,4-oxadiazole-3-yl)-butyl]-théophylline;
la 7-[4-(5-[2-diéthylaminoéthyl]-1,2,4-oxadiazole-3-yl)-butyl]-théophylline;
la 7-[5-(5-méthyl-1,2,4-oxadiazole-3-yl)-pentyl]-théophylline; et
la 7-[5-(5-[2-diéthylaminoéthyl]-1,2,4-oxadiazole-3-yl)-pentyl]-théophylline.

7. Procédé de préparation des dérivés de la théophylline de formule I selon l'une des revendications 1 à 6, caractérisé en ce que:

(A) on fait réagir des amidoximes de formule générale II

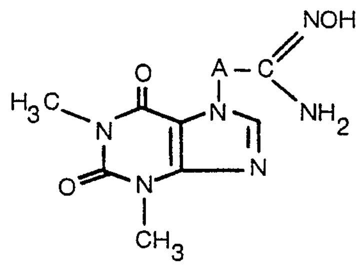

(II)

dans laquelle A a les significations indiquées dans la revendication 1, avec des acides de formule générale III

$$R_6—COOH \qquad (III)$$

dans laquele $R_6$ a les mêmes significations que le symbole $R_1$ de la revendication 1 ou représente un groupe convertible en un groupe $R_1$, et/ou avec des dérivés acylants des acides de formule générale III, avec formation directe de composés de formule générale Ic

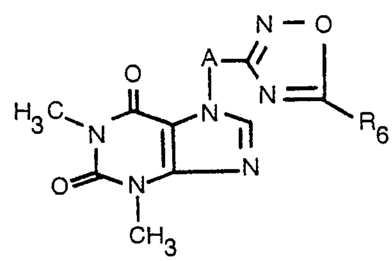

(Ic)

dans laquelle A et $R_6$ ont les significations indiquées ci-dessus, après quoi, lorsque c'est nécessaire, on convertit $R_6$ en $R_1$ ou bien

(B) On fait réagir des amidoximes de formule générale II avec des acides de formule générale III ou des dérivés acylants des acides de formule générale III avec formation de dérivés acylés de formule générale IV

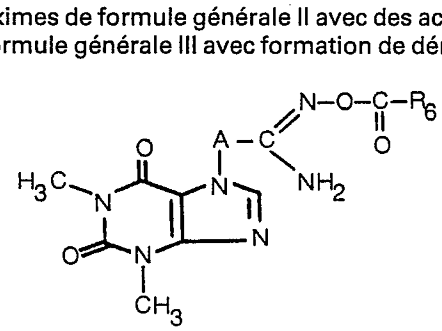

(IV)

dans laquelle A et $R_6$ ont les significations indiquées ci-dessus, et on cyclise les composés de formule générale IV, éventuellement après les avoir isolés, avec élimination d'eau, après quoi, lorsque c'est nécessaire, on convertit $R_6$ en $R_1$; ou bien

(C) on fait réagir des composés de formule générale V

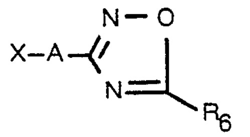

(V)

dans laquelle A et $R_6$ ont les significations indiquées ci-dessus et X représente un halogène ou un groupe ester d'acide sulfonique, avec la théophylline en présence d'un catalyseur basique ou avec un sel de la théophylline et d'une base, et lorsque c'est nécessaire, on convertit $R_6$ en $R_1$ ou bien

(D) on fait réagir des oléfines de formule générale VI

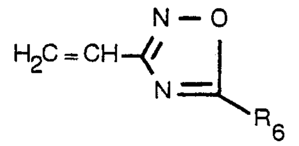

(VI)

dans laquelle $R_6$ a les significations indiquées ci-dessus, en présence d'un catalyseur basique, avec la théophylline, ce qui donne des composés de formule générale Id

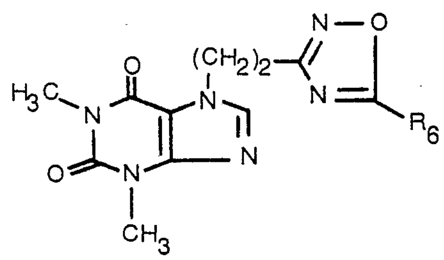

(Id)

dans laquelle $R_6$ a les significations indiquées ci-dessus, après quoi, lorsque c'est nécessaire, on convertit $R_6$ en $R_1$, ou bien

(E) on fait réagir des époxydes de formule générale VII

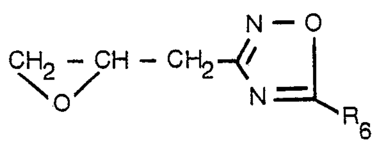

(VII)

dans laquelle $R_6$ a les significations indiquées ci-dessus, avec la théophylline, ce qui donne un composé de formule I dans laquelle

A représente

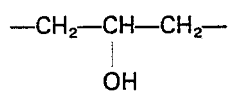

et lorsque c'est nécessaire, on convertit $R_6$ en $R_1$.

8. Procédé selon la revendication 7, pour préparer les composés de formule générale I dans laquelle $R_1$ représente un groupe amino-alkyle de formule générale 1 de la revendication 1 ou de formule

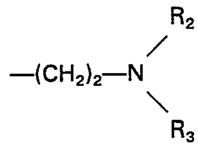

dans laquelle $R_2$ et $R_3$ ont les significations indiquées dans la revendication 1, caractérisé en ce que

(a) on fait réagir des composés de formules générales I ou IV dans lesquelles $R_6$ représente un groupe vinyle, 2-éthoxyéthyle, halogénoalkyle en C1—C4, O-méthane-sulfonylalkyle ou O-toluène-sulfonylalkyle et A a les significations indiquées dans la revendication 1, avec une amine de formule générale $HNR_2R_3$ dans laquelle $R_2$ et $R_3$ ont les significations indiquées dans la revendication 1, ou bien

(b) on soumet à condensation réductrice des composés de formules générales I ou IV dans lesquelles

$R_6$ représente un groupe oxoalkyle en C1—C4 et A a les significations indiquées dans la revendication 1, avec une amine de formule générale $R_8NH_2$ dans laquelle $R_8$ a les significations indiquées pour $R_2$, à l'exception de l'hydrogène, ou bien

(c) on alkyle des composés de formule générale I ou IV dans laquelle $R_6$ représente $—(CH_2)_n—NH_2$ dans laquelle n est égal à 1, 2, 3 ou 4 et A a les significations indiquées dans la revendication 1.

9. Procédé selon la revendication 7 ou 8 caractérisé en ce que l'on utilise des composés de formules III, IV, V, VI ou VII dans lesquelles $R_6$ représente un groupe vinyle, 2-éthoxyéthyle, oxoalkyle en C3—C4, O-toluène-sulfonylalkyle, O-méthane-sulfonylalkyle ou halogénoalkyle.

10. Procédé selon la revendication 9, caractérisé en ce que l'on utilise des composés de formule IV dans laquelle $R_6$ représente un groupe chloralkyle ou bromalkyle.

11. Médicaments caractérisés en ce qu'ils contiennent en tant que substances actives, avec des produits auxiliaires et/ou véhicules usuels, des composés de formule gégérale I selon l'une des revendications 1 à 6.

12. Médicaments selon la revendication 11, caractérisés en outre en ce qu'ils contiennent d'autres substances possédant une activité biologique.

**Claims**

1. 7-Substituted theophylline derivatives of general formula I

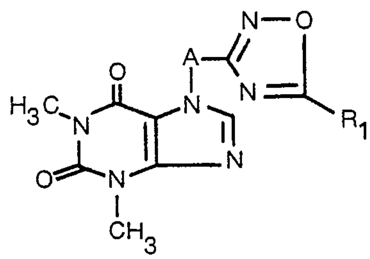

(I),

wherein

A represents straight chained or branched $C_{1-5}$ alkylene or

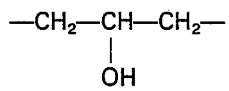

and

$R_1$ represents hydroxy, straight chained or branched $C_{1-10}$ alkyl, halolalkyl or hydroxyalkyl, $C_{5-6}$ cycloalkyl, vinyl, 2-ethoxyethyl, $C_{2-5}$ carbonylalkyl, benzyl, 2,2-diphenylethyl, theophylline-7-ylmethyl, an aminoalkyl group of general formula 1

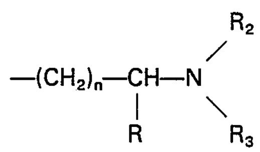

(1)

or a phenyl group of general formula 2

$$R_4R_5C_6H_3— \quad (2)$$

whilst in formula 1 or 2

R represents H or methyl,

$R_2$ and $R_3$ represent hydrogen or straight chained or branched $C_{1-4}$ alkyl or

$R_2$ and $R_3$ together with the nitrogen atom may form a 5—6 membered ring, which may optionally contain a nitrogen or oxygen atom optionally substituted by methyl,

n represents 0, 1, 2 or 3 and

$R_4$ and $R_5$ may independently represent H, chlorine, hydroxy, methoxy, ethoxy, methyl or amino, and the physiologically acceptable acid addition salts thereof.

2. Compounds of general formula I as claimed in claim 1, wherein

A represents $C_{1-3}$ alkylene or

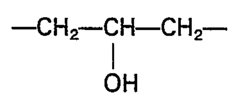

and

$R_1$ represents $C_{1-4}$ alkyl or an aminoalkyl group of general formula 1, and the acid addition salts thereof.

3. 7-[(5-methyl-1,2,4-oxadiazol-3-yl)-methyl]-theophylline,
7-[(5-ethyl-1,2,4-oxadiazol-3-yl)-methyl]-theophylline,
7-[(5-[propan-2-yl]-1,2,4-oxadiazol-3-yl)-methyl]-theophylline,
7-[(5-[butan-1-yl]-1,2,4-oxadiazol-3-yl)-methyl]-theophylline,
7-[(5-[2-methyl-propan-1-yl]-1,2,4-oxadiazol-3-yl)-methyl]-theophylline,
7-[(5-[1,1-dimethyl-ethan-1-yl]-1,2,4-oxadiazol-3-yl)-methyl]-theophylline,
7-[(5-[pentyl]-1,2,4-oxadiazol-3-yl)-methyl]-theophylline,
7-[(5-[decyl]-1,2,4-oxadiazol-3-yl)-methyl]-theophylline,
7-[(5-cyclopentyl-1,2,4-oxadiazol-3-yl)-methyl]-theophylline,
7-[(5-cyclohexanyl-1,2,4-oxadiazol-3-yl)-methyl]-theophylline,
7-[(5-[1-hydroxy-ethyl]-1,2,4-oxadiazol-3-yl)-methyl]-theophylline,
7-[(5-chloromethyl-1,2,4-oxadiazol-3-yl)-methyl]-theophylline,
7-[(5-[3-chloropropyl]-1,2,4-oxadiazol-3-yl)-methyl]-theophylline,
7-[(5-[oxobutyl]-1,2,4-oxadiazol-3-yl)-methyl]-theophylline,
7-[(5-[2-chloroethyl]-1,2,4-oxadiazol-3-yl)-methyl]-theophylline,
7-[(5-[2-ethoxy-ethyl]-1,2,4-oxadiazol-3-yl)-methyl]-theophylline,
7-[(5-isopropylaminomethyl-1,2,4-oxadiazol-3-yl)-methyl]-theophylline,
7-[(5-[2-N-methylpiperazinoethyl]-1,2,4-oxadiazol-3-yl)-methyl]-theophylline,
7-[(5-[2-pyrrolidinoethyl]-1,2,4-oxadiazol-3-yl)-methyl]-theophylline,
7-[(5-[3-piperidinopropyl]-1,2,4-oxadiazol-3-yl)-methyl]-theophylline,
7-[(5-[3-diethylaminopropyl]-1,2,4-oxadiazol-3-yl)-methyl]-theophylline,
7-[(5-[3-isopropylaminobutyl]-1,2,4-oxadiazol-3-yl)-methyl]-theophylline,
7-[(5-[4-diethylaminobutyl]-1,2,4-oxadiazol-3-yl)-methyl]-theophylline,
7-[(5-phenyl-1,2,4-oxadiazol-3-yl)-methyl]-theophylline,
7-[(5-[4-chlorophenyl]-1,2,4-oxadiazol-3-yl)-methyl]-theophylline,
7-[(5-[2-hydroxyphenyl]-1,2,4-oxadiazol-3-yl)-methyl]-theophylline,
7-[(5-hydroxy-1,2,4-oxadiazol-3-yl)-methyl]-theophylline,
7-[(5-diethylaminomethyl-1,2,4-oxadiazol-3-yl)-methyl]-theophylline,
7-[(5-piperidinomethyl-1,2,4-oxadiazol-3-yl)-methyl]-theophylline,
7-[(5-aminomethyl-1,2,4-oxadiazol-3-yl)-methyl]-theophylline,
7-[(5-dimethylaminomethyl-1,2,4-oxadiazol-3-yl)-methyl]-theophylline,
7-[(5-[2-isopropylaminoethyl]-1,2,4-oxadiazol-3-yl)-methyl]-theophylline,
7-[(5-[2-piperidinoethyl]-1,2,4-oxadiazol-3-yl)-methyl]-theophylline,
7-[(5-[2-morpholinoethyl]-1,2,4-oxadiazol-3-yl)-methyl]-theophylline,
7-[(5-[2-diethylaminoethyl]-1,2,4-oxadiazol-3-yl)-methyl]-theophylline,
7-[(5-benzyl-1,2,4-oxadiazol-3-yl)-methyl]-theophylline, and
7-[(5-[theophylline-7-ylmethyl]-1,2,4-oxadiazol-3-yl)-methyl]-theophylline.

4. 7-[2-(5-Methyl-1,2,4-oxadiazol-3-yl)-ethyl]-theophylline;
7-[2-(5-ethyl-1,2,4-oxadiazol-3-yl)-ethyl]-theophylline;
7-[2-(5-pentyl-1,2,4-oxadiazol-3-yl)-ethyl]-theophylline;
7-[2-(5-cyclohexyl-1,2,4-oxadiazol-3-yl)-ethyl]-theophylline;
7-[2-(5-(2-chloroethyl)-1,2,4-oxadiazol-3-yl)-ethyl]-theophylline;
7-[2-(5-chloromethyl-1,2,4-oxadiazol-3-yl)-ethyl]-theophylline;
7-[2-(5-[3-chloropropyl]-1,2,4-oxadiazol-3-yl)-ethyl]-theophylline;
7-[2-(5-[1-hydroxyethyl]-1,2,4-oxadiazol-3-yl)-ethyl]-theophylline;
7-[2-(5-diethylaminomethyl-1,2,4-oxadiazol-3-yl)-ethyl]-theophylline;
7-[2-(5-[2-isopropylaminoethyl]-1,2,4-oxadiazol-3-yl)-ethyl]-theophylline;
7-[2-(5-[2-pyrrolidinoethyl]-1,2,4-oxadiazol-3-yl)-ethyl]-theophylline;
7-[2-(5-[2-piperidinoethyl]-1,2,4-oxadiazol-3-yl)-ethyl]-theophylline;
7-[2-(5-[2-morpholinoethyl]-1,2,4-oxadiazol-3-yl)-ethyl]-theophylline;
7-[2-(5-[2-diethylaminoethyl]-1,2,4-oxadiazol-3-yl)-ethyl]-theophylline;
7-[2-(5-[2-N-methylpiperazinoethyl]-1,2,4-oxadiazol-3-yl)-ethyl]-theophylline;
7-[2-(5-[3-piperidinopropyl]-1,2,4-oxadiazol-3-yl)-ethyl]-theophylline;
7-[2-(5-[3-diethylaminopropyl]-1,2,4-oxadiazol-3-yl)-ethyl]-theophylline;
7-[2-(5-phenyl-1,2,4-oxadiazol-3-yl)-ethyl]-theophylline;
7-[2-(5-hydroxy-1,2,4-oxadiazol-3-yl)-ethyl]-theophylline;
7-[2-(5-[4-chlorophenyl]-1,2,4-oxadiazol-3-yl)-ethyl]-theophylline;
7-[2-(5-[2-hydroxyphenyl]-1,2,4-oxadiazol-3-yl)-ethyl]-theophylline;
7-[2-(5-benzyl-1,2,4-oxadiazol-3-yl)-ethyl]-theophylline and
7-[2-(5-[2,2-diphenylethyl]-1,2,4-oxadiazol-3-yl)-ethyl]-theophylline.

5. 7-[3-(5-Methyl-1,2,4-oxadiazol-3-yl)-propyl]-theophylline;
7-[3-(5-cyclohexyl-1,2,4-oxadiazol-3-yl)-propyl]-theophylline;

7-[3-(5-[2-chloroethyl]-1,2,4-oxadiazol-3-yl)-propyl]-theophylline;
7-[3-(5-2-piperidinoethyl]-1,2,4-oxadiazol-3-yl)-propyl]-theophylline;
7-[3-(5-[2-diethylaminoethyl]-1,2,4-oxadiazol-3-yl)-propyl]-theophylline;
7-[3-(5-[2-morpholinoethyl]-1,2,4-oxadiazol-3-yl)-propyl]-theophylline;
7-[3-(5-[2-isopropylaminoethyl]-1,2,4-oxadiazol-3-yl)-propyl]-theophylline;
7-[3-(5-[2-N-methylpiperazinoethyl]-1,2,4-oxadiazol-3-yl)-propyl]-theophylline;
7-[3-(5-[3-piperidinopropyl]-1,2,4-oxadiazol-3-yl)-propyl]-theophylline;
7-[3-(5-hydroxy-1,2,4-oxadiazol-3-yl)-propyl]-theophylline;
7-[3-(5-phenyl-1,2,4-oxadiazol-3-yl)-propyl]-theophylline;
7-[2-hydroxy-3-(5-methyl-1,2,4-oxadiazol-3-yl)-propyl]-theophylline;
7-[2-hydroxy-3-(5-ethyl-1,2,4-oxadiazol-3-yl)-propyl]-theophylline;
7-[2-hydroxy-3-(5-cyclohexyl-1,2,4-oxadiazol-3-yl)-propyl]-theophylline;
7-[2-hydroxy-3-(5-n-propyl-1,2,4-oxadiazol-3-yl)-propyl]-theophylline;
7-[2-hydroxy-3-(5-[2-diethylaminoethyl]-1,2,4-oxadiazol-3-yl)-propyl]-theophylline;
7-[2-hydroxy-3-(5-[2-piperidinoethyl]-1,2,4-oxadiazol-3-yl)-propyl]-theophylline;
7-[2-hydroxy-3-(5-[2-morpholinoethyl]-1,2,4-oxadiazol-3-yl)-propyl]-theophylline;
7-[2-hydroxy-3-(5-[2-N-methylpiperizinoethyl]-1,2,4-oxadiazol-3-yl)-propyl]-theophylline;
7-[2-hydroxy-3-(5-[2-isopropylaminoethyl]-1,2,4-oxadiazol-3-yl)-propyl]-theophylline;
7-[2-hydroxy-3-(5-phenyl-1,2,4-oxadiazol-3-yl)-propyl]-theophylline;
7-[2-hydroxy-3-(5-[2-hydroxyphenyl]-1,2,4-oxadiazol-3-yl)-propyl]-theophylline;
7-[2-hydroxy-3-(5-hydroxy-1,2,4-oxadiazol-3-yl)-propyl]-theophylline;
7-[3-(5-benzyl-1,2,4-oxadiazol-3-yl)-propyl]-theophylline and
7-[3-(5-[2,2-diphenylethyl]-1,2,4-oxadiazol-3-yl)-propyl]-theophylline.

6. 7-[4-(5-Methyl-1,2,4-oxadiazol-3-yl)-butyl]-theophylline;
7-[4-(5-[2-piperidinoethyl]-1,2,4-oxadiazol-3-yl)-butyl]-theophylline;
7-[4-(5-[2-diethylaminoethyl]-1,2,4-oxadiazol-3-yl)-butyl]-theophylline;
7-[5-(5-methyl-1,2,4-oxadiazol-3-yl)-pentyl]-theophylline and
7-[5-(5-[2-diethylaminoethyl]-1,2,4-oxadiazol-3-yl)-pentyl]-theophylline.

7. Process for the preparation of the theophylline derivatives of general formula I as claimed in one of claims 1 to 6, characterised by

a) reacting an amidoxime of general formula II

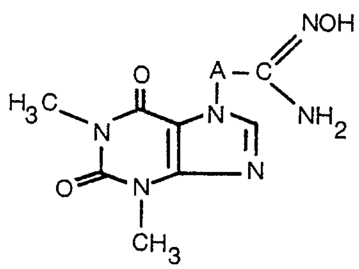

(II)

(wherein A is as defined in claim 1) with an acid of general formula III

$$R_6—COOH \qquad (III)$$

wherein $R_6$ is the same as $R_1$ in claim 1 or represents a group which can be converted into $R_1$, and/or with a derivative of the acid of formula III suitable for acylation in order to produce directly a compound of general formula Ic

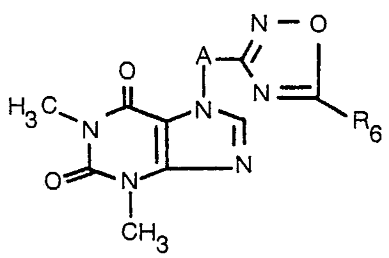

(Ic)

wherein A and $R_6$ are defined as above and if necessary converting $R_6$ into $R_1$, or

b) reacting an amidoxime of general formula II with an acid of general formula III or a derivative thereof suitable for acylation — wherein $R_6$ is as defined above, in order to produce an acylated derivative of general formula IV

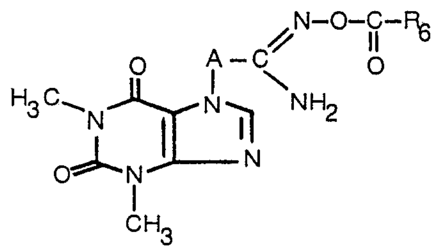

(IV)

wherein A and $R_6$ are as defined above, and cyclising the compound of general formula IV, optionally after isolating said compound whilst eliminating water, and if necessary converting $R_6$ into $R_1$, or

c) reacting a compound of general formula V

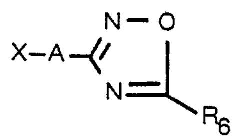

(V)

(wherein A and $R_6$ are as defined above and X represents halogen or a sulfonic acid ester group) with theophylline in the presence of a basic catalyst or with a salt of theophylline formed with a base, and if necessary converting $R_6$ into $R_1$ or

d) reacting an olefin of general formula VI

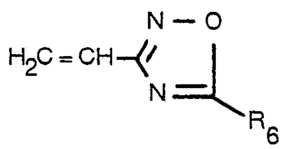

(VI)

(wherein $R_6$ is as given above) with theophylline in the presence of a basic catalyst in order to produce compounds of general formula Id

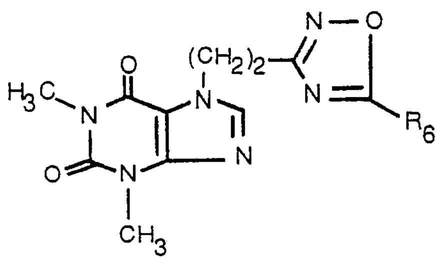

(Id)

(wherein $R_6$ is as defined above) and if necessary converting $R_6$ into $R_1$, or

e) reacting an epoxide of general formula VII

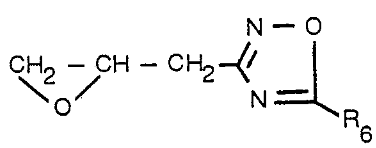

(VII)

(wherein $R_6$ is as defined above) — with theophylline to obtain a compound of formula I wherein

A = 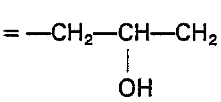

and if necessary converting $R_6$ into $R_1$.

8. A process as claimed in claim 7 in order to produce compounds of general formula I wherein $R_1$ represents an aminoalkyl group of general formula I as in claim 1 or

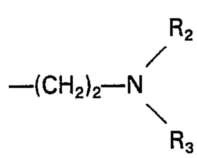

wherein $R_2$ and $R_3$ are defined as in claim 1, characterised by

(a) reacting a compound of general formula I or IV wherein $R_6$ represents vinyl, 2-ethoxyethyl, $C_{1-4}$ haloalkyl, O-mesylalkyl or O-tosylalkyl, and A is defined as in claim 1, with an amine of general formula $HNR_2R_3$ (wherein $R_2$ and $R_3$ are as defined in claim 1) or

(b) reductive condensation of a compound of general formula I or IV, wherein $R_6$ represents $C_{1-4}$ oxoalkyl, and A is as defined in claim 1, with an amine of general formula $R_8NH_2$ (wherein $R_8$ has the same meanings as $R_2$, with the exception of hydrogen) or

(c) alkylating a compound of general formula I or IV wherein $R_6$ represents $-(CH_2)_n-NH_2$ (wherein n represents 1, 2, 3 or 4) and A is as defined in claim 1.

9. Process as claimed in claim 7 or 8, characterised by the use of compounds of formulae III, IV, V, VI or VII, wherein $R_6$ represents vinyl, 2-ethoxyethyl, $C_{3-4}$ oxoalkyl, O-tosylalkyl, O-mesylalkyl or haloalkyl.

10. Process as claimed in claim 9, characterised by the use of compounds of formula IV wherein $R_6$ represents chloroalkyl or bromoalkyl.

11. Pharmaceutical compositions, characterised by compounds of general formula I as claimed in one of claims 1 to 6 as active substances together with conventional excipients and/or carriers.

12. Pharmaceutical compositions as claimed in claim 11, further characterised by a content of other biologically active substances.